(19) Europäisches Patentamt / European Patent Office / Office européen des brevets

(11) **EP 4 748 855 A1**

(12) **EUROPEAN PATENT APPLICATION**


(43) Date of publication: **27.05.2026 Bulletin 2026/22**

(21) Application number: **24215209.8**

(22) Date of filing: **25.11.2024**

(51) International Patent Classification (IPC): ***C07K 14/705*** *(2006.01)*

(52) Cooperative Patent Classification (CPC): **C07K 14/70596; C07K 14/70503; C12N 9/2497; G01N 33/80;** C07K 2319/30; C07K 2319/32; C07K 2319/70; C12Y 302/02006; G01N 2333/70596; G01N 2800/24

(84) Designated Contracting States: **AL AT BE BG CH CY CZ DE DK EE ES FI FR GB GR HR HU IE IS IT LI LT LU LV MC ME MK MT NL NO PL PT RO RS SE SI SK SM TR**
Designated Extension States: **BA**
Designated Validation States: **GE KH MA MD TN**



(71) Applicant: **mAB-Factory GmbH**
**38108 Braunschweig (DE)**

(72) Inventors:
- **Hust, Michael**
  **38118 Braunschweig (DE)**
- **Steinke, Stephan**
  **38444 Wolfsburg (DE)**
- **Gnann, Dianne Celine**
  **38104 Braunschweig (DE)**
- **Garritsen, Hendrikus S.P.**
  **38116 Braunschweig (DE)**

(74) Representative: **Fuchs Patentanwälte Partnerschaft mbB**
**Tower 185**
**Friedrich-Ebert-Anlage 35-37**
**60327 Frankfurt am Main (DE)**


Remarks:
The complete document including Reference Table(s) and the Sequence Listing(s) can be downloaded from the EPO website

(54) **NEW MOLECULES FOR THE PREVENTION OF FALSE POSITIVE AGGLUTINATION**


(57) Disclosed are fusion proteins for the prevention of false positive agglutination as well as methods of production, uses and kits thereof. (Figure 7)

Patient sample: + + + + + + + + +
CD38-mFc: - - - + + + - - -
N1-N2B-mFc: - - - - - - + + +
Cell type: I II III I II II I II III
1 2 3 4 5 6 7 8 9


FIG. 7

EP 4 748 855 A1

## Description

Disclosed are fusion proteins for the prevention of false positive agglutination as well as methods of production, uses and kits thereof. The disclosed fusion proteins are in particular characterized by non-interference with Duffy-antibodies, thus, preventing hemolytic transfusion-reactions in Fy(a)-positive patients.

### Background

The mission of the blood group serology laboratory is the determination of blood group characteristics on the erythrocytes and the discovery of antibodies against foreign blood group characteristics in the plasma of a blood sample. The aim of all efforts is to provide serologically compatible blood products. In the case of erythrocyte concentrations, the serological tolerability is determined by a non-reactive ("negative") tolerance test, called cross-matching test. In this cross-matching test a recipient's blood plasma is contacted with a donor's red blood cells. If the plasma contains antibodies against structures on the donor's blood cells, visible clumping or destruction of the red blood cells (RBCs) occurs.

Illnesses can cause changes that interfere with these examinations. Leukemias cause blood group characteristics to disappear, diseases that are associated with increased concentrations of plasma proteins (e.g., fibrinogen, $\alpha$2-macroglobulin or IgM), promote the formation of so-called money rolls in vitro and thereby simulate agglutinations and autoimmune hemolysis caused by antibodies that are reactive in cross-matching tests.

In addition to these illness-related disorders, there are also disorders caused by medications. Drugs can stimulate the immune system to produce autoantibodies, which then also bind to the patient's erythrocytes and, if in excess produced, are present freely in the plasma and cause the antibody search test and tolerance tests to be positive. In some of these patients, the autoantibodies cause drug-dependent immune hemolysis.

Drugs like daratumumab, isatuximab (SAR-650984), MOR202 and TAK079 which all target CD38, are used for the treatment of multiple myeloma, raised the problem to another level, since patients treated for example with daratumumab are always positive in cross-matching tests because CD38 is present on many blood cell types, including RBCs. Basically, daratumumab, isatuximab (SAR-650984), MOR202 and TAK079 cause false positive reactions in cross-matching tests and render it nearly impossible for patients to receive blood transfusions for lack of a reliable compatibility test. This is particularly problematic, since this patient group needs regular blood-transfusions as part of their therapy.

A similar complicated situation occurs for drugs like magrolimab (Hu5F9-G4) or CC-90002, which bind to CD47, which is also displayed on the surface of RBCs. Combining anti-CD47 antibodies with standard chemotherapy can enhance the immune system's ability to target cancer cells weakened by chemotherapy. Further, drugs like pembrolizumab (anti-PD-1) or nivolumab (anti-PD-1) are sometimes used in combination with anti-CD47 therapies to provide a dual immune-boosting effect by both allowing T cells to attack tumors and promoting macrophage phagocytosis.

In 2016, the German Society for transfusion-medicine and immunohematology (DGTI) published two techniques in order to prevent positive reactions in cross-matching tests with patients treated with therapeutic antibodies, such as daratumumab: (1) Treatment of the test cells (blood-cells) with dithiothreitol (DTT) to destroy the CD38 and/or CD47 antigen and/or (2) providing the patient with "antigenically compatible erythrocytes". However, these attempts had several disadvantages. One of them was that DTT has been shown to also destroy other proteins on the erythrocyte surface, including transfusion relevant antigens of the Kell, Lutheran and further blood groups. DTT has additionally been shown to be ineffective in mitigating the interference caused by anti-CD47 antibodies.

Another technology relies on trypsin-treatment of the donor RBC, removing most of the surface markers, which of course may mask other, dangerous cross-reactivities between recipient and donor. An additional proposed technology involves the absorption of anti-CD38 antibodies in patient samples by using whole cells or cell lysate containing CD38 antigens.

In 2018 a new product was developed called DaraEx®, which is daratumumab cleaved with pepsin, resulting in F(ab')2-fragments, which are then incubated with the blood-sample before the cross-matching-test is performed. The idea is to mask all CD38-receptors with the truncated daratumumab-version in order to prevent cross-reactivity during cross-matching. Unfortunately, despite efforts to develop a similar solution for CD47, no product in this respect is available on the market.

However, in any case, the DaraEx®-method is quite expensive, exceeding several folds the costs for normal cross-matching tests. For example, the current costs exceed the revenue that can be achieved in Germany for cross-matching according to the fee schedule.

Recombinant, soluble CD38-fusion proteins (sCD38) were developed in order to provide cost-effective alternatives.

In a clinical environment, blocking the CD38 antigens on reagent or donor cell or the neutralization of the anti-CD38 antibodies in patient samples prior to testing are considered the most suitable. When focusing on automation of blood compatibility testing processes the neutralization in the patient samples is advisable.

Similar approaches have been tested for CD47, although not successful. The only commercially available substance is TTI-621 (SIRPαFc) which is a fusion protein which includes the extracellular domain of SIRPα and may indirectly affect the action of anti-CD47 therapies by blocking the CD47-SIRPα interaction, although it does not serve as a soluble CD47 itself.

Furthermore, some of these soluble proteins for "baiting" therapeutic anti-CD38 and/or anti-CD47 antibodies were reported to interfere with Duffy antibody tests. The Duffy antigen (also called Fy, DARC or CD234) is a protein found on the surface of red blood cells and is significant in the context of blood transfusions. If blood containing Duffy antigens is transfused into a patient who has Duffy antibodies, an immune reaction can occur. This can cause hemolysis, or even more serious complications, like kidney failure or disseminated intravascular coagulation (DIC). To prevent such reactions, blood needs to be carefully matched for Duffy antigens and antibodies before transfusion. Thus, the reports of prior art methods comprising sCD38 interfering with Duffy antibody tests raised significant concerns.

Thus, a need existed to provide new strategies in order to prevent false positive agglutination mediated by the cross-reaction of an anti-CD38-antibodywith CD38 and/or anti-CD47-antibody with CD47, and to ensure at the same time that there is no interference with Duffy antigen tests.

Details of invention

The disclosure identified a new class of fusion-proteins suitable for the prevention of false positive agglutination in-vitro or in a subject and which does not interfere with Duffy-antigen tests.

In a **first aspect** the disclosure relates to a fusion protein for binding to false positive agglutinating antibody, comprising an extracellular domain of CD38 and/or an extracellular domain CD47, a linker region, a hinge-region, and a Fc-fragment.

In one embodiment, the extracellular domain of CD38 may exist in various forms, influenced by several factors, including alternative splicing, post-translational modifications, genetic mutations, proteolytic cleavage, fusion with other proteins, and cell-specific contexts.

In one embodiment the extracellular domain of CD38 may be an isoform of CD38 generated by alternative splicing of the CD38, in one embodiment the extracellular domain of CD38 comprises at least isoform 1 and isoform 2 of CD38. Additionally, in another embodiment the extracellular domain of CD38 may comprise post-translational modifications such as glycosyla-tions, phosphorylation and/or disulfide bond formations. In one embodiment, the extracellular domain may differ in structure depending on the cellular context, as CD38 is expressed on various cell types, including B cells, Tcells, and NK cells, with potential variations in post-translational modifications or isoform expression. In yet another embodiment the extracellular domain of CD38 also comprises CD38 homologs, such as CD157. In one embodiment, the extracellular domain of CD38 is selected from the group consisting of SEQ ID NO.1 and 2.

In one embodiment, the extracellular domain of CD47 may exist in various forms. CD47, also known as integrin-associated protein (IAP), is a transmembrane protein that plays a crucial role in various cellular processes, including immune response, cell adhesion, and apoptosis. In one embodiment, the extracellular domain of CD47 according to SEQ ID NO. 3.

Both CD38 as well as CD47 are present on the surface of red blood cells (RBC).

In yet another embodiment the fusion protein according to the present disclosure may comprise both the extracellular domain of CD38 and of CD47. This fusion protein provides a double function and simplifies the cross-reaction tests, because blocking of both therapeutic antibodies (if potentially present in a patient sample) may be blocked within one test run without the need to do two separate tests.

At the same time the further structure of the fusion protein provides no interference with any tests directed to the Duffy-antigens.

In one embodiment, the linker region is selected from the group consisting of SEQ ID NO. 4 and/or 5. In one embodiment also variants of SEQ ID. No. 4 and/or 5 with up to one amino acid difference in form of a point-mutation, deletion and/or insertion are encompassed. In one embodiment the linker may be omitted.

In one embodiment, the hinge-region may be selected from the hinge-region of IgG, IgA, and IgD, as well as variants thereof. The hinge-region may also be of animal origin, such as a murine origin. In one embodiment, the hinge-region is selected from the group consisting of SEQ ID NO. 6, 7, 8, and/or 9. In one embodiment also variants of SEQ ID NO. 6, 7, 8, and/or 9with up to two amino acid difference in form of a point-mutation, deletion and/or insertion are encompassed.

In one embodiment, the Fc-region may be derived from IgG (Immunoglobulin G), IgM (Immunoglobulin M), IgA (Immunoglobulin A), IgD (Immunoglobulin D), and IgE (Immunoglobulin E), as well as variants thereof. The Fc-region may be of animal origin, such as a murine IgG rather than of human IgG, to avoid any possible interactions with anti-human IgG antibodies during purification and testing. In one embodiment the murine Fc of IgG2a may be used. In one embodiment, the Fc-region is selected from the group consisting of SEQ ID NO. 10, 11, 12, 13, 14, 15, 16, 17 and/or 18.

In one embodiment, the structural elements of the fusion protein of the present disclosure enhance solubility, and/or improve the ability to purify the fusion protein during production.

In one embodiment the fusion protein may have at least 95%, at least 96%, at least 97%, at least 98% at least 99%, or at least 99,5% sequence identity to a sequence selected from the group consisting of SEQ ID NO 19 to 342. In one embodiment the sequence variability of the fusion protein may be different in different parts of the molecule, such as that the fusion protein may have sequence similarity to a sequence selected from the group consisting of SEQ ID NO 19 to 342

- with up to two amino-acids difference, up to one amino-acid difference, or no amino-acid difference (i.e. sequence 100% identity), in the hinge and/or linker region,
- at least 94%, at least 95%, at least 96%, at least 97%, at least 98% at least 99%, or at least 99,5% sequence identity in the fc-domain, and
- at least 90%, at least 91%, at least 92%, at least 93%, at least 94% at least 95%, or at least 96% amino-acid difference in the extracellular CD38-domain and/or extracellular CD47-domain.

In one embodiment the fusion protein is identical to a sequence selected from the group consisting of SEQ ID NO 19 to 342.

In one embodiment the fusion protein prevents false positive agglutination in a blood-sample when added with at least a 7-fold molar excess as compared to the false positive agglutinating antibody, with at least a 6-fold molar excess as compared to the false positive agglutinating antibody, with at least a 5-fold molar excess as compared to the false positive agglutinating antibody, or with at least a 4-fold molar excess as compared to the false positive agglutinating antibody. Optionally, the fusion protein prevents false positive agglutination in a blood-sample when added with at most a 40-fold molar excess as compared to the false positive agglutinating antibody, with at most a 30-fold molar excess as compared to the false positive agglutinating antibody, with at most a 20-fold molar excess as compared to the false positive agglutinating antibody, or with at most a 10-fold molar excess as compared to the agglutinating antibody.

Optionally, the fusion protein prevents false positive agglutination in a blood-sample when added with an amount of from 3-fold to 45-fold molar excess as compared to the false positive agglutinating antibody, with an amount of from 4-fold to 40-fold molar excess as compared to the false positive agglutinating antibody, with an amount of from 5-fold to 30-fold molar excess as compared to the false positive agglutinating antibody, or with an amount of from 6-fold to 20-fold molar excess as compared to the false positive agglutinating antibody.

In one embodiment the fusion protein prevents false positive agglutination mediated by the cross-reaction of an anti-CD38-antibody with CD38 and/or anti-CD47-antibody with CD47 in a blood-sample when added to the blood sample. As such the final concentration of the fusion protein in the blood sample in order to prevent false positive agglutination is 100,000 nM or less, 75,000 nM or less, 65,000 nM or less, or 50,000 nM or less.

In one embodiment the fusion protein has at least one of the following characteristics:

- it has an EC50-value of at least 0.20 nM;
- it prevents false positive agglutination mediated by the cross-reaction of an anti-CD38-antibody with CD38 and/or anti-CD47-antibody with CD47 in a blood-sample when added with at least a 3-fold molecular excess as compared to the false positive agglutinating antibody;
- it prevents false positive agglutination mediated by the cross-reaction of an anti-CD38-antibody with CD38 and/or anti-CD47-antibody with CD47 in a blood-sample when added with at most 45-fold molecular excess as compared to the false positive agglutinating antibody;
- it prevents false positive agglutination mediated by the cross-reaction of an anti-CD38-antibody with CD38 and/or anti-CD47-antibody with CD47 in a blood-sample when being present in the blood-sample in a concentration of 100,000 nM or less.

In a **second aspect** the disclosure relates to an analytical or pharmaceutical composition comprising the disclosed fusion protein. An "analytical composition" is defined herein as comprising the fusion protein as lyophilizate or as soluble protein in water in conjunction with other excipients, such as a buffer, stabilizer, anti-freezing agent, or the like, which may be used to conduct in-vitro analysis, tests and measurements, in particular cross-matching tests with blood-samples as part of blood compatibility testing. A "pharmaceutical composition" is defined herein as any formulations designed for the administration of drugs or therapeutic agents to patients. These compositions typically contain both active and inactive ingredients, formulated to ensure the safety, efficacy, and stability of the drug. The specific components and formulation depend on the type of drug, the intended route of administration, and other factors. Examples are: tablets and capsules, injectable solutions (including intravenous (IV), intramuscular (IM), and subcutaneous (SC) routes), oral liquids, or the like. In particular, in the prevention and/or treatment of false positive agglutination-events in a subject or patient, such as Acute Hemolytic Transfusion Reaction (AHTR), Delayed Hemolytic Transfusion Reaction (DHTR), Febrile Non-Hemolytic Transfusion Reaction (FNHTR), Transfusion-Related Acute Lung Injury (TRALI), and/or Transfusion-Associated Graft vs. Host Disease (TA-GVHD).

The analytical or pharmaceutical composition comprising the fusion protein according to the present disclosure may further comprise at least one compound selected from the group consisting of a buffer, a stabilizer, a diluent, a filler, a binder, a disintegrant, a glidant, a preservative, a solvent, a surfactant, as well as any combinations thereof.

In one embodiment, the disclosure relates to a nucleic acid encoding for the disclosed fusion protein, such as an expression vector comprising the nucleic acid sequences in order to express the fusion protein as well as, optionally, further elements, such as promotor-elements, ORI-elements, terminator-sequence, selection markers, fusion-tags, spacer/linker-sequences, poly-A-signal, or combinations thereof.

In a **third aspect,** the disclosure relates to the use of the fusion protein according to the specification, or the composition as disclosed hereinunder, for the prevention of agglutination mediated by the cross-reaction of an anti-CD38-antibody with CD38 and/or anti-CD47-antibody with CD47 in a blood-sample.

In one embodiment uses are encompassed wherein the blood-sample of the recipient and/or donor cannot be excluded of being positive for the presence of antibodies against at least one of the antigens selected from the group consisting of $Fy^a$ (Duffy a), $Fy^b$ (Duffy b), $Fy^3$, $Fy^4$, $Fy^5$, $Fy^6$, and $Fy^x$, as well as combinations thereof.

In one embodiment uses are encompassed wherein the blood-sample of the recipient and/or donor cannot be excluded of being positive for the presence of antibodies against CD38 and/or CD47.

One embodiment related to the fusion protein according to the disclosure for use in a method of treating a patient, or the composition disclosed hereinunder for use in a method of treating a patient, wherein the treatment comprises

- taking a sample volume from the patient, such as blood, plasma or serum,
- testing the sample volume for compatibility with a donor sample, the donor sample comprising red blood cells, wherein the testing comprises contacting the fusion protein or the composition with the sample volume or the donor sample, and combining the sample volume with the donor sample,
- administering a blood donation of the donor to the patient in case of compatibility,

wherein said patient or said donor cannot be excluded of having antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof; and/or

wherein said patient or said donor is exhibiting expression of at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof.

One further embodiment related to the fusion protein according to the disclosure for use in a method of treating a patient, or the composition disclosed hereinunder for use in a method of treating a patient, wherein the treatment comprises

- taking a sample volume from the patient, such as blood, plasma or serum,
- testing the sample volume for compatibility with a donor sample, the donor sample comprising red blood cells, wherein the testing comprises contacting the fusion protein or the composition with the sample volume or the donor sample, and combining the sample volume with the donor sample,
- administering a blood donation of the donor to the patient in case of compatibility,

wherein said patient or said donor cannot be excluded of having antibodies against CD38 and/or CD47 as well as combinations thereof; and/or

wherein said patient or said donor cannot be excluded of having antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof; and/or

wherein said patient or said donor is exhibiting expression of at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof.

In yet another embodiment the fusion protein or composition for use in the method of treating a patient as disclosed encompasses a use, wherein said patient or donor is positive for antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof and/or wherein said patient or donor is positive for antibodies against CD38 and/or CD47.

In yet another embodiment the fusion protein or composition for use in the method of treating a patient as disclosed encompasses a use, wherein the patient has received treatment with anti-CD38-antibodies and/or anti-CD47-antibodies.

One embodiment relates to the fusion protein according to the disclosure for use in a method of treating a patient, or the composition according the disclosure for use in a method of treating a patient, wherein the fusion protein or composition is administered to a patient having a condition selected from Acute Hemolytic Transfusion Reaction (AHTR), Delayed Hemolytic Transfusion Reaction (DHTR), Febrile Non-Hemolytic Transfusion Reaction (FNHTR), Transfusion-Related Acute Lung Injury (TRALI), and/or Transfusion-Associated Graft vs. Host Disease (TA-GVHD) caused by a incompatible blood donation comprising antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof; or antigens selected from $Fy^a$ (Duffy a), $Fy^b$ (Duffy b), $Fy^3$, $Fy^4$, $Fy^5$, $Fy^6$, and $Fy^x$, as well as combinations thereof.

In one embodiment the disclosure relates to the use of the fusion protein according to the present disclosure, or a composition comprising the same, for the prevention of agglutination in a blood-sample, in particular in blood samples wherein the recipient serum comprises anti-CD38-antibody and/or anti-CD47 antibodies, and/or anti-Duffy-antigen antibodies and the donor blood sample comprises CD38 and/or CD47 as well as at least one Duffy-antigen.

In one embodiment the disclosure relates to the use of the fusion protein according to the present disclosure, or a composition comprising the same, for the prevention of agglutination in a blood-sample, in particular in blood samples wherein the recipient serum comprises anti-CD38-antibody and/or anti-Duffy-antigen antibodies and the donor blood sample comprises CD38 as well as at least one Duffy-antigen.

In one embodiment the disclosure relates to the use of the fusion protein according to the present disclosure, or a composition comprising the same, for the prevention of agglutination in a blood-sample, in particular in blood samples wherein the recipient serum comprises anti-CD47 and/or anti-Duffy-antigen antibodies and the donor blood sample comprises CD47 as well as at least one Duffy-antigen.

In the context of blood agglutination, "Duffy" refers to a blood group system that includes antigens found on the surface of red blood cells. The most common and clinically significant antigens in the Duffy system are $Fy^a$ or $Fy^b$. These antigens are encoded by the FY gene. If an individual has antibodies against the Duffy antigens, their blood will agglutinate when mixed with red blood cells that express the corresponding antigen. This is important in blood transfusion and can lead to hemolytic transfusion reactions if mismatched blood is transfused. The Duffy antigens are also receptors for the malaria parasite *Plasmodium vivax.* Individuals who lack Duffy antigens on their red blood cells are resistant to infection by this parasite. The prevalence of Duffy antigens varies among different populations. For instance, $Fy^a$ (Duffy a), $Fy^b$ (Duffy b) are common in people of European descent, while the absence of Duffy antigens is more common in people of African descent. Matching Duffy antigens is crucial in blood transfusion to avoid agglutination reactions. Duffy antibodies can cross the placenta and cause hemolytic disease in a newborn if there is an incompatibility between the mother and fetus. Certain molecules which are used to prevent agglutination based on anti-CD38-reactions, and/or anti-CD47-reactions, "mask" Duffy antigen tests, thereby resulting in false Duffy-negative results although the blood-donor is positive for one or more anti-Duffy antibodies (cf. also Figure 12 and description of Figure 12).

Thus, in one embodiment the use of the fusion protein according to the present disclosure, or a composition comprising the same, pertains to the use in an agglutination test, wherein the blood-sample of the recipient may comprise both therapeutic anti-CD38 antibodies, such as daratumumab, and/or therapeutic anti-CD47 antibodies, such as magrolimab, as well as anti-Duffy alloantibodies. In yet another embodiment the use of the fusion protein according to the present disclosure, or a composition comprising the same, pertains to the use in an agglutination test, wherein the blood-sample of the donor may comprise CD38 and/or CD47 antigen as well as Duffy-antigens. In yet another embodiment the use of the fusion protein according to the present disclosure, or a composition comprising the same, pertains to the use in an agglutination test, wherein the blood-sample of the recipient may comprise both therapeutic anti-CD38 antibodies, such as daratumumab, and/or therapeutic anti-CD47 antibodies, such as magrolimab, as well as anti-Duffy alloantibodies and wherein the blood-sample of the donor may comprise both CD38 antigen as well as Duffy-antigens.

Thus, in one embodiment, the disclosure relates to the use of the fusion protein according to the present disclosure, or a composition comprising the same, wherein the blood-sample of the recipient comprises antibodies against at least one of the antigens selected from the group consisting of $Fy^a$ (Duffy a), $Fy^b$ (Duffy b), $Fy^3$, $Fy^4$, $Fy^5$, $Fy^6$, and $Fy^x$, as well as combinations thereof. In another embodiment, the disclosure relates to the use of the fusion protein according to the present disclosure, or a composition comprising the same, wherein the blood-sample of the donor comprises at least one of the antigens selected from the group consisting of $Fy^a$ (Duffy a), $Fy^b$ (Duffy b), $Fy^3$, $Fy^4$, $Fy^5$, $Fy^6$, and $Fy^x$, as well as combinations thereof.

In one embodiment, the disclosure relates to the use of the fusion protein according to the present disclosure, or a composition comprising the same, for the treatment of a patient with a blood-sample donation, wherein said patient group cannot be excluded of having antibodies against CD38 and/or CD47, and/or antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof. In another embodiment, the disclosure relates to the use of the fusion protein according to the present disclosure, or a composition comprising the same, for testing agglutination of a donor blood-sample with a recipient blood-sample, wherein said donor blood-sample cannot be excluded of having CD38 and/or at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof.

In one embodiment, the disclosure relates to the use of the fusion protein according to the present disclosure, or a composition comprising the same, for the treatment of a patient with a blood donation, wherein said patient group is selected by being positive for antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof.

In one embodiment, the disclosure relates to the use of the fusion protein according to the present disclosure, or a composition comprising the same, for the treatment of a patient having anti-CD38-antibodies and/or anti-CD47-antibodies.

In one embodiment, the disclosure relates to the use of the fusion protein according to the present disclosure, or a pharmaceutical composition comprising the same, for use as a medicament.

In a **fourth aspect** the present disclosure relates to a method for specifically neutralizing or blocking the binding of an anti-CD38 antibody and/or anti-CD47 antibody in a sample, comprising:

a) providing a volume of the sample containing the anti-CD38 antibody and/or the anti-CD47 antibody; and

b) incubating the volume of the sample with a volume of the composition according to the present disclosure, containing the fusion protein in an amount sufficient to neutralize the anti-CD38 antibody and/or the anti-CD47 antibody in the sample.

In one embodiment the disclosure relates to the use of the disclosed fusion protein for a cross-matching analysis of a blood transfusion. In one embodiment the method or use comprises the steps of

a) Providing a blood sample from a donor;

b) providing an acceptor blood sample;

c) adding composition of fusion protein to donor blood sample or the acceptor blood sample, and/or the mix of donor blood sample and the acceptor blood sample;

d) evaluate whether agglutination has occurred.

Such cross-matching tests may comprise a major crossmatch, which involves testing the compatibility between the recipient's serum/plasma and the donor's red blood cells (RBCs) for ABO and RhD blood group systems. The goal is to ensure that the donor's RBCs do not agglutinate (clump) when mixed with the recipient's serum/plasma and vice versa. Optionally, cross-matching tests may comprise a minor crossmatch which involves testing the compatibility between the recipient's RBCs and the donor's plasma for non-ABO and non-RhD blood group antigens. This is done to identify potential antibodies in the donor's plasma that could react with antigens on the recipient's RBCs. Agglutination or hemolysis in the minor crossmatch suggests the presence of antibodies in the donor's plasma that could react with antigens on the recipient's RBCs.

In one embodiment the fusion protein of the present disclosure is used in the disclosed methods to prevent agglutination in a starting concentration from 5 to 50 mg/ml, from 10 to 40 mg/ml, or from 15 to 35 mg/ml, resulting in a target concentration within the reaction solution from 1-10 mg/ml, from 1.5-8 mg/ml or from 2-5 mg/ml.

In one embodiment the fusion protein prevents agglutination in a blood-sample when added to the blood sample. As such the final concentration of the fusion protein in the blood sample in order to prevent agglutination is 100,000 nM or less, 75,000 nM or less, 65,000 nM or less, or 50,000,000 nM or less. In yet another embodiment the final concentration of the fusion protein in the blood sample in order to prevent agglutination is at least 4,000 nM, at least 6,000 nM, at least 10,000 nM, or at least 20,000 nM. In one embodiment the fusion protein prevents agglutination in a blood-sample when present in the blood sample in an amount of from 4,000 nM to 100,000 nM, in an amount of from 5,000 nM to 75,000 nM, in an amount of from 6,000 nM to 60,000 nM, or in an amount of from 10,000 nM to 50,000 nM.

In one embodiment the amount of fusion protein in the blood sample is from 100-to 300 μg, from 120-to 240 μg, from 140-to 220 μg, or from 160-to 200 μg.

The term "specifically" as used above, is defined as only neutralizing or blocking anti-CD38 antibodies and/or anti-CD47 antibodies, but no other antibodies in the sample of the recipient, in particular no other allopathic antibodies, in particular no anti-Duffy-antigen antibodies.

In one embodiment the sample is selected from the group consisting of blood, plasma, and serum, as well as combinations thereof.

In one embodiment the sample comprises daratumumab, isatuximab (SAR-650984) and/or MOR202.

In one embodiment the volume of the sample is at least 25 µl, at least 50 µl, at least 100 µl, at least 200 µl, at least 250 µl, or at least 300 µl. In one embodiment the volume of the sample is 500 µl or less, 450 µl or less, 400 µl or less, or 350 µl or less. In one embodiment the volume of the sample ranges from about 25 µl to about 500 µl, from about 50 µl to about 450 µl, or from about 100 µl to about 300 µl.

In one embodiment the volume of the composition is at least 0.5 µl, at least 1 µl, at least 2 µl, at least 5 µl, at least 10 µl, or at least 20 µl. In one embodiment the volume of the composition is 50 µl or less, 45 µl or less, 40 µl or less, or 35 µl or less. In one embodiment the volume of the composition ranges from about 0.5 µl to about 50 µl, from about 1 µl to about 45 µl from about 5 µl to about 40 µl, or from about 10 µl to about 35 µl.

In one embodiment the concentration of the anti-CD38 antibody in the sample is at least 0.005 µg/ml, at least 0.05 µg/ml, at least 0.5 µg/ml, at least 5 µg/ml, at least 50 µg/ml, or at least 500 µg/ml. In one embodiment the concentration of the anti-CD38 antibody in the sample is 2000 µg/ml or less, 1750 µg/ml or less, 1500 µg/ml or less, or 1000 µg/ml or less. In one embodiment the concentration of the anti-CD38 antibody in the sample ranges from about 0.005 µg/ml to about 2000 µg/ml, from about 0.05 µg/ml to about 1750 µg/ml, from about 0.5 µg/ml to about 1500 µg/ml, or from about 5 µg/ml to about 1000 µg/ml.

In one embodiment the concentration of the fusion protein in the composition is at least 1 mg/ml, at least 5 mg/ml, at least 10 mg/ml, at least 15 mg/ml, at least 20 mg/ml, or at least 25 mg/ml. In one embodiment the concentration of the fusion protein in the composition is 400 mg/ml or less, 350 mg/ml or less, 300 mg/ml or less, 250 mg/ml or less, 200 mg/ml or less, or 150 mg/ml or less. In one embodiment the concentration of the fusion protein in the composition ranges from about 1 mg/ml to about 400 mg/ml, from about 1 mg/ml to about 400 mg/ml, from about 1 mg/ml to about 400 mg/ml, or from about 1 mg/ml to about 400 mg/ml.

In one embodiment the neutralizing effect of the fusion protein is at least 10%, at least 20%, at least 30%, at least 40%, at least 50%, at least 60%, at least 70% or at least 80%. In one embodiment the neutralizing effect of the fusion protein is 100% or less, 99% or less, 98% or less, 95% or less, 90% or less, 88% or less, 85% or less, or 82% or less. In one embodiment the neutralizing effect of the fusion protein ranges from about 10% to about 100%, from about 20% to about 99%, from about 30% to about 95%, from about 40% to about 90%, or from about 50% to about 88%.

The term "neutralizing effect" is defined herein as to the percentage of antibody which, upon binding to fusion protein according to the disclosure, is not able to mediate any false positive agglutination. For example, if 70% of all antibodies in the sample are not able to bind to the CD38 and/or CD47 on the RBCs, due to being already bound to the "bait body", i.e. the fusion protein of the present disclosure, then the neutralizing effect of the fusion protein is 70%.

In one embodiment the binding activity of the anti-CD38 antibody and/or anti-CD47 antibody is selected from the group consisting of interference with pre-transfusion blood transfusion tests, interference with blood compatibility tests, and interference with antibody therapy.

In a **fifth aspect** the present disclosure also relates to a kit for bio-monitoring research and diagnostic assays, comprising the fusion protein according to the disclosure, or the composition according to the disclosure, a plate and reagents for identifying the presence of antibodies.

A composition of 15-30 mg/mL of a fusion protein with an amino acid sequence selected from Seq ID No. 19 to 342 in solution or as a lyophilizate for the treatment of samples prior to column agglutination testing, solid phase testing or further blood compatibility testing methods. Further embodiments are the integration of a fusion protein with an amino acid sequence selected from Seq ID No. 19 to 342 into Ready-to-use columns or into the reagents of the solid phase testing method for the purpose of the automatization of blood compatibility testing.

The invention is further exemplified by the following embodiments.

In one embodiment the disclosure relates to a fusion protein for binding to an agglutinating antibody, comprising

an extracellular domain of CD38 and/or CD47, as well as fragment thereof,

optionally, a linker region,

a hinge-region,

and an Fc-fragment.

In one embodiment the sequence of the extracellular domain of CD38, and/or a fragment thereof, is selected from SEQ ID. No. 1 or 2 and the extracellular domain of CD47, and/or a fragment thereof, is selected from SEQ ID. No. 3.

In one embodiment the linker region comprises the sequence according to SEQ ID. No. 4 or 5, as well as variants thereof with up to one amino acid difference in form of a point-mutation, deletion and/or insertion as compared to SEQ ID.

No. 4 or 5.

In one embodiment the hinge-region comprises the sequence according to SEQ ID. No. 6 to 9, as well as variants thereof with up to two amino acids difference in form of a point-mutation, deletion and/or insertion as compared to SEQ ID. No. 6 to 9.

In one embodiment the sequence of the Fc fragment has at least 98% sequence identity to a sequence selected from the group consisting of SEQ ID. No. 10 to 18. In one embodiment the sequence of the Fc fragment is identical to a sequence selected from the group consisting of SEQ ID. No. 10 to 18.

In one embodiment the sequence of the fusion protein has at least 97% sequence identity to a sequence selected from the group consisting of SEQ ID. No. 19 to 342. In one embodiment the sequence of the fusion protein is identical to a sequence selected from the group consisting of SEQ ID. No. 19 to 342.

In one embodiment the sequence of the fusion protein has at least 97% sequence identity to SEQ ID. No. 55. In one embodiment the sequence of the fusion protein has at least 97% sequence identity to SEQ ID. No. 57.

In one embodiment the sequence of the fusion protein has 100% sequence identity to SEQ ID. No. 55. In one embodiment the sequence of the fusion protein has 100% sequence identity to SEQ ID. No. 57.

In one embodiment the fusion protein has at least one of the following characteristics:

- it has an EC50-value of 0.35 nM or less;
- it prevents agglutination mediated by the cross-reaction of an anti-CD38 with CD38 and/or anti-CD47-antibody with CD47 in a blood-sample when added with at least an equimolar ratio as compared to the agglutinating antibody;
- it prevents agglutination mediated by the cross-reaction of an anti-CD38 with CD38 and/or anti-CD47-antibody with CD47 in a blood-sample when added with at most 45-fold molar excess as compared to the agglutinating antibody;
- it prevents agglutination mediated by the cross-reaction of an anti-CD38 with CD38 and/or anti-CD47-antibody with CD47 in a blood-sample when present in the blood-sample at a concentration of 100,000 nM or less.

In one embodiment the disclosure relates to an analytical, diagnostic or pharmaceutical composition comprising the fusion protein as disclosed hereinunder, further comprising at least one compound selected from the group consisting of a buffer, a stabilizer, a diluent, a filler, a binder, a disintegrant, a glidant, a preservative, a solvent, a surfactant, as well as any combinations thereof.

In one embodiment the disclosure relates to the use of a fusion protein as disclosed hereinunder, or the composition as disclosed hereinunder, for the prevention of agglutination mediated by the cross-reaction of an anti-CD38 with CD38 and/or anti-CD47-antibody with CD47 in a blood-sample.

In one embodiment the disclosure relates to the use, wherein the blood-sample of the recipient and/or donor cannot be excluded of being positive for the presence of antibodies against at least one of the antigens selected from the group consisting of $Fy^a$ (Duffy a), $Fy^b$ (Duffy b), $Fy^3$, $Fy^4$, $Fy^5$, $Fy^6$, and $Fy^x$, as well as combinations thereof.

In one embodiment the present disclosure relates to the use, wherein the blood-sample of the recipient and/or donor cannot be excluded of being positive for the presence of antibodies against CD38 and/or CD47.

In one embodiment the present disclosure relates to a fusion protein according to the disclosure for use in a method of treating a patient, or the composition according to the disclosure for use in a method of treating a patient, wherein the treatment comprises

- taking a sample volume from the patient, such as blood, plasma or serum,
- testing the sample volume for compatibility with a donor sample, the donor sample comprising red blood cells, wherein the testing comprises contacting the fusion protein or the composition with the sample volume or the donor sample, and combining the sample volume with the donor sample,
- administering a blood donation of the donor to the patient in case of compatibility,

wherein said patient or said donor cannot be excluded of having antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof; and/or wherein said patient or said donor is exhibiting expression of at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof.

In one embodiment the disclosure relates to the fusion protein or composition for use in the method of treating a patient according to the disclosure, wherein said patient or donor is positive for antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof.

In one embodiment the present disclosure relates to the fusion protein or composition for use in the method of treating a patient a disclosed herein, wherein the patient has received treatment with anti-CD38-antibodies and/or anti-CD47-antibodies.

In one embodiment the present disclosure relates to the use in a method of treating a patient, or the composition as disclosed herein for use in a method of treating a patient, wherein the fusion protein or composition is administered to a patient having, a condition selected from Acute Hemolytic Transfusion Reaction (AHTR), Delayed Hemolytic Transfusion Reaction (DHTR), Febrile Non-Hemolytic Transfusion Reaction (FNHTR), Transfusion-Related Acute Lung Injury (TRALI), and/or Transfusion-Associated Graft vs. Host Disease (TA-GVHD) caused by a incompatible blood donation comprising antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof; or antigens selected from $Fy^a$ (Duffy a), $Fy^b$ (Duffy b), $Fy^3$, $Fy^4$, $Fy^5$, $Fy^6$, and $Fy^x$, as well as combinations thereof.

In one embodiment the present disclosure relates to a method for neutralizing or blocking the binding of an anti-CD38 and/or an anti-CD47 antibody in a sample, comprising:

providing a volume of the sample, consisting of blood, plasma, and serum, containing the anti-CD38 and/or CD47 antibody; and

incubating the sample with the fusion protein disclosed hereinunder or a volume of the composition disclosed hereinunder, comprising an amount of the fusion protein sufficient to specifically neutralize the anti-CD38 and/or CD47 antibody in the sample.

In one embodiment the present disclosure relates to a method, wherein the sample is from a patient who has undergone treatment with an anti-CD38 such as daratumumab, isatuximab (SAR-650984), MOR202 and/or TAK079, and/or anti-CD47 antibody, such as magrolimab (Hu5F9-G4) and/or CC-90002.

In one embodiment the present disclosure relates to a kit for bio-monitoring research and diagnostic assays, comprising the fusion protein disclosed hereinunder, or the composition disclosed hereinunder, a plate and reagents for identifying the presence of antibodies.

Definitions

"CD38" is defined herein as a transmembrane glycoprotein and enzyme expressed on various cell types, including immune cells such as B cells, T cells, and natural killer (NK) cells, as well as on plasma cells and certain tumor cells. It catalyzes the conversion of NAD+ into cyclic ADP-ribose (cADPR) and ADP-ribose, playing a crucial role in calcium signaling, which influences cell proliferation, differentiation, and apoptosis.

In oncology, particularly in multiple myeloma, CD38 is highly expressed on malignant plasma cells, making it a valuable therapeutic target. Monoclonal antibodies such as daratumumab and isatuximab are designed to bind to CD38, inducing immune-mediated destruction of these cells through mechanisms like complement-dependent cytotoxicity (CDC) and antibody-dependent cellular cytotoxicity (ADCC). Thus, CD38 serves as a critical molecule in both normal immune function and the pathogenesis of certain cancers, with significant therapeutic implications.

"CD47" is defined herein as a transmembrane glycoprotein that plays a vital role in regulating cell interactions and signalling within the immune system. It is widely expressed on various cell types, including red blood cells, immune cells, and many tumour cells. CD47 functions primarily as a phagocytosis inhibiting signal by binding to the SIRP$\alpha$ receptor on macrophages and allowing cells to evade immune detection.

In oncology, CD47 is often overexpressed in various cancers, including hematologic malignancies and solid tumours, making it an attractive therapeutic target. Monoclonal antibodies targeting CD47, such as magrolimab, are developed to block the CD47-SIRP$\alpha$ interaction, thereby promoting phagocytosis of cancer cells by macrophages and enhancing the immune response against tumours. As such, CD47 represents a critical component in cancer immunotherapy, facilitating the recognition and elimination of malignant cells.

An "antigen" is a molecule or molecular structure that is recognized by the immune system as foreign or non-self. Antigens can stimulate an immune response, leading to the production of antibodies or activation of immune cells. Antigens are often found on the surface of pathogens such as bacteria, viruses, and fungi, as well as on the surface of abnormal or foreign cells, such as cancer cells.

The term "sequence identity", in the context of this disclosure and in the context of amino acids, refers to the degree of similarity between the amino acid sequences of two proteins or peptides. It is a measure of how many amino acids in the sequences are identical when aligned.

In bioinformatics and molecular biology, scientists often compare the amino acid sequences of different proteins to understand their structural and functional relationships. The measure of sequence identity is expressed as a percentage and is calculated by dividing the number of identical amino acids by the total number of amino acids in the shorter of the two sequences, and then multiplying by 100.

The formula for sequence identity is:

$$\text{Sequence Identity} = \left(\frac{\text{Number of Identical Amino Acids}}{\text{Total Number of Amino Acids in the Shorter Sequence}}\right) \times 100$$

For example, if two protein sequences are aligned, and out of 100 amino acids, 80 are found to be identical, the sequence identity would be

$$\left(\frac{80}{100}\right) \times 100 = 80\%.$$

Sequence identity is a crucial metric in bioinformatics because it provides insights into the evolutionary relationships between proteins. Proteins with higher sequence identity are more likely to share a common ancestry and may have similar structures and functions. Sequence identity may be used to classify proteins into families, predict protein function, and infer evolutionary relationships.

An "erythrocyte cell" is a specialized type of blood cell, commonly known as a red blood cell. Erythrocytes are a crucial component of the blood and play a vital role in transporting oxygen from the lungs to the rest of the body tissues and bringing carbon dioxide back to the lungs for exhalation.

A "tumor cell" is a cell that has undergone uncontrolled and abnormal growth, leading to the formation of a lump or mass of tissue called a tumor. Tumors can be benign or malignant. Tumor cells can originate from various cell types in the body, and they can be classified based on their tissue of origin. For example, if tumor cells arise from epithelial tissue, the tumor is termed a carcinoma. If the cells originate from connective tissues such as bone or muscle, the tumor is referred to as a sarcoma.

The term "Fc-region" or ""Fc-domain" refers to the "fragment crystallizable" region, which is a portion of an antibody molecule that plays a crucial role in mediating various effector functions of the immune system. There are several classes and subclasses of antibodies, each with a distinct Fc region. The main antibody classes in humans are IgG, IgM, IgA, IgD, and IgE.

IgG antibodies are the most abundant class of antibodies in the bloodstream. The Fc region of IgG antibodies plays a key role in interactions with immune cells and complement proteins. IgG antibodies have subclasses, including IgG1, IgG2, IgG3, and IgG4, each with slightly different Fc structures.

IgM antibodies are the first antibodies produced during an immune response. They are often found on the surface of B cells. The Fc region of IgM is involved in complement activation and immune cell binding.

IgA antibodies are found in mucosal areas, such as the respiratory and gastrointestinal tracts. IgA antibodies have two subclasses, IgA1 and IgA2, each with a distinct Fc region. IgA is involved in mucosal immunity.

IgD antibodies are found on the surface of B cells and are involved in the activation of these cells. The Fc region of IgD is less studied compared to other antibody classes.

IgE antibodies are involved in allergic reactions and immune responses against parasitic infections. The Fc region of IgE is responsible for binding to receptors on mast cells and basophils.

The term "hinge region" refers to a flexible or unstructured segment of a protein structure that allows for movement or flexibility between different domains or regions of the protein. This region acts like a hinge, enabling conformational changes and flexibility in the overall structure of the protein. Hinge regions are often found in proteins with multiple functional domains or subunits. In the context of antibodies the term "hinge region" is commonly used to describe a specific flexible segment of the antibody structure. The hinge region is the flexible connector between the two arms (Fab fragments) and the stem (Fc fragment).

The term "click chemistry" used to describe a set of chemical reactions that are efficient, selective, and modular. These reactions are characterized by their reliability, simplicity, and the ability to generate high yields under mild conditions. The concept of click chemistry was introduced by chemist K. Barry Sharpless in 2001. One of the most well-known click chemistry reactions is the copper(I)-catalyzed azide-alkyne cycloaddition (CuAAC). In this reaction, an azide group reacts with an alkyne group to form a triazole ring, and copper(I) is used as a catalyst to enhance the reaction rate. The CuAAC reaction has found widespread applications in the fields of chemistry, biology, and materials science, including the labeling and modification of biomolecules, synthesis of polymers, and construction of bioconjugates.

The "SpyTag/SpyCatcher system" is a protein tagging system based on a covalent isopeptide bond formation between two small peptide sequences: SpyTag and SpyCatcher. This system was developed as a tool for site-specific and irreversible protein-protein conjugation. It was introduced by the research group of Mark Howarth. SpyTag is a short peptide sequence (13 amino acids) that can be genetically fused to the protein of interest. It contains an exposed reactive site that forms an isopeptide bond with SpyCatcher. SpyCatcher is a larger protein (approximately 15 kDa) that can be expressed and purified separately. It contains a reactive site complementary to SpyTag. The reactive site consists of an amino acid sequence that reacts specifically with the SpyTag. When SpyTag and SpyCatcher are mixed, they rapidly form a covalent bond between the SpyTag reactive site and the SpyCatcher reactive site. This bond is similar to the sort of bond formed between proteins and their targets in the biological world.

"Maleimide chemistry" involves the reaction between maleimide groups and thiol groups, typically found in cysteine residues of proteins. This technique is widely used for site-specific conjugation.

"NHS chemistry" involves the reaction between NHS esters and primary amines in proteins. NHS esters are often used to modify lysine residues on proteins.

"Copper-free click chemistry" involves bioorthogonal reactions that do not require copper as a catalyst. For example, strain-promoted azide-alkyne cycloaddition (SPAAC) is a copper-free variant of click chemistry.

Similar to maleimide chemistry, "thiol-maleimide conjugation" involves the reaction between thiol groups and maleimide groups. This reaction is selective and can be used for site-specific conjugation.

"Sortase-Mediated Conjugation": Sortase is an enzyme that recognizes a specific amino acid sequence (LPXTG) and catalyzes the cleavage of the peptide bond between threonine and glycine. This system is used for site-specific conjugation of proteins or peptides containing the LPXTG motif.

"Staudinger ligation" involves the reaction between azides and triarylphosphines, resulting in the formation of an amide bond. This bio-orthogonal reaction is commonly used for protein labeling.

"Photoaffinity labeling" involves incorporating a photoreactive group into a biomolecule. Upon exposure to light, the photoreactive group forms a covalent bond with nearby molecules. This technique is used for studying protein-protein interactions.

"Genetic code expansion" involves incorporating unnatural amino acids with specific chemical handles into proteins during their synthesis. These handles can then be used for selective bio-conjugation reactions.

"Enzymatic ligation" involves the use of enzymes to catalyze the formation of covalent bonds between biomolecules. For example, transglutaminase can catalyze the formation of amide bonds between proteins.

"Blood group system" refers to one or more red blood cell surface antigens, which are encoded by a single gene or closely linked genes and are thus categorized together. These antigens result from site-specific polymorphisms in functionally diverse membrane proteins, glycoproteins and glycoproteins and can be recognized by alloantibodies.

The term "agglutination" is used in this disclosure to refer to the clumping of red blood cells mediated by antibodies binding to red blood cells surface antigens and the detection thereof resulting in a positive test during blood compatibility testing. This detection may be carried out using column agglutination testing, solid phase testing or further blood compatibility testing methods.

"False positive agglutination" is used in this disclosure to describe the agglutination mediated by therapeutic antibodies targeting and binding an antigen which is also found on the surface of red blood cells. The resulting positive tests do not reflect the presence of an alloantibody against transfusion-relevant blood group antigens and thus represent false positives in the context of blood compatibility testing. Certain drugs (e.g. Daratumumab) may may interfere with the diagnostic detection of allo/auto erythrocyte antibodies through "false positive agglutination" reactions.

The term "prevent false positive agglutination" means herein that an antibody which usually would bind specifically to one of the at least two antigens in the fusion protein, when present in a blood-sample, e.g., CD38 on an erythrocyte, and its binding result in a false positive agglutination, is not able to exert any agglutination within the blood-sample anymore. For example, a scale from 0 to 4 may be used, where 0 represents no agglutination, and 4 represents strong agglutination. In such a case "prevent false positive agglutination" means that the agglutination is of grade 0 or 1, in one embodiment of grade 0. In one embodiment "prevent false positive agglutination" is met when 5000 nM or less of the fusion protein in the final test sample of the blood sample result in a grade of 1, and/or even 0. Such a prevention of "prevent false positive agglutination" may also prevent "hemolysis", thus the term "prevent false positive agglutination" encompasses also "prevent hemolysis".

"Hemolysis" refers to the destruction or rupture of red blood cells (erythrocytes), resulting in the release of hemoglobin into the surrounding fluid, typically plasma. Hemolysis can occur under various conditions and may have different causes. When red blood cells are damaged or destroyed, hemoglobin, the iron-containing molecule responsible for oxygen transport, is released into the bloodstream.

The "EC50 (effective concentration 50%)" is defined herein as the concentration of a drug or a ligand needed to produce a response halfway between the baseline and the maximum response in a biological system. In other words, it is the concentration at which a drug or compound is exerting 50% of its maximum effect. In a typical dose-response curve, the x-axis represents the concentration of the drug or ligand, and the y-axis represents the biological response. The EC50 is the concentration at which the response is halfway between the baseline (minimum response) and the maximum response. In the context of false positive agglutination, the term EC50 refers to 50% effective concentration of the fusion protein of the disclosure which prevents false positive agglutination. In the present disclosure the affinity based on the half maximal effective concentration needed of the fusion protein of the present disclosure to bind to the respective target therapeutic anti-CD38-antibody and/or anti-CD47 monoclonal antibody is defined as "EC50" hereinunder. In one embodiment the EC50 is at least 0.20 nM, at least 0.25 nM, at least 0.30 nM, at least 0.35 nM, at least 0.40 nM, or at least 0.50 nM. In one embodiment the EC50-value is at most 1.00 nM, at most 0.95 nM, at most 0.85 nM at most 0.75 nM, at most 0.70 nM, or at most 0.60 nM. In one embodiment the EC50-value is from 0.20 nM to 1.00 nM, or from 0.25 nM to 0.85 nM.

"Acute Hemolytic Transfusion Reaction (AHTR)" is a severe and potentially life-threatening reaction that occurs when incompatible blood is transfused, leading to the rapid destruction of donor red blood cells. Symptoms may include fever, chills, back pain, hemoglobinuria, and acute renal failure.

"Delayed Hemolytic Transfusion Reaction (DHTR)" occurs days to weeks after a transfusion. It involves a slower destruction of transfused red blood cells and may lead to anemia.

"Febrile Non-Hemolytic Transfusion Reaction (FNHTR)" is characterized by fever and chills and may occur as a response to white blood cell antibodies, cytokines, or other components in the transfused blood. It is usually not life-threatening but can cause discomfort for the patient.

"Transfusion-Related Acute Lung Injury (TRALI)" is a rare but serious reaction characterized by acute respiratory distress and lung injury. It is thought to be associated with antibodies in donor plasma.

"Transfusion-Associated Graft vs. Host Disease (TA-GVHD)" is a rare but often fatal complication where viable T lymphocytes in the transfused blood attack the recipient's tissues. It is more common in immunocompromised patients.

The term "baitbody" is used herein as a synonym for the fusion protein of the disclosure able to bind specifically to anti-CD38-antibody and/or anti-CD47 antibodies.

Description of the Figures

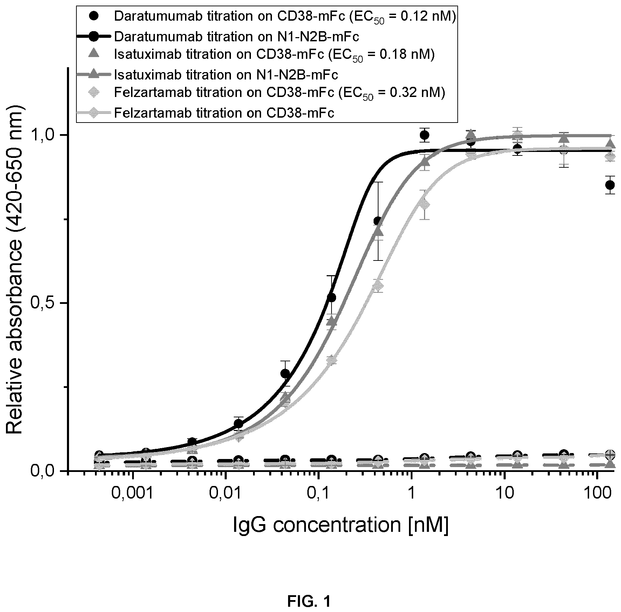

**Figure 1** - Results of titration ELISA of anti-CD38 IgGs on the CD38-mFc baitbody. The relative absorbance (420-650nm) correlating the binding intensity was plotted against the IgG concentration in nM. The bound daratumumab isatuximab and felzartamab (MOR202) to immobilized baitbody were detected with a goat anti-human IgG-peroxidase (A0170). The N1-N2B-mFc baitbody was used as a control for the binding specificity. All steps were carried out in 2% MPBST at room temperature (20°C).

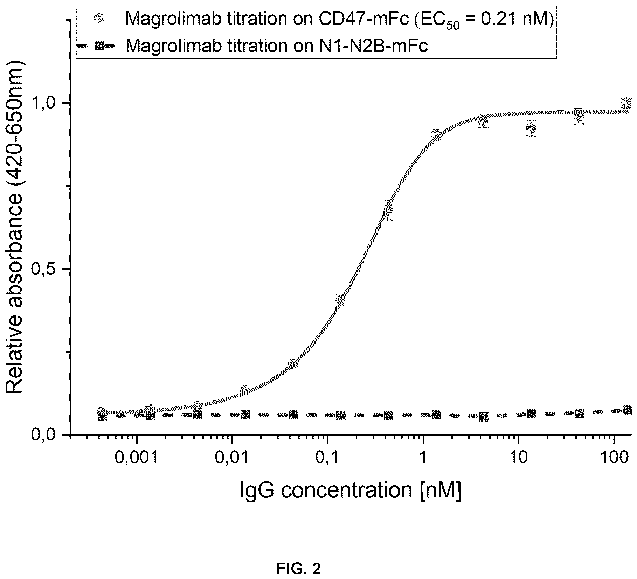

**Figure 2** - Results of titration ELISA of magrolimab on the CD47-mFc baitbody. The relative absorbance (420-650nm) correlating the binding intensity was plotted against the IgG concentration in nM. The bound magrolimab to immobilized baitbody were detected with a goat anti-human IgG-peroxidase (A0170). The N1-N2B-mFc baitbody was used as a control for the binding specificity. All steps were carried out in 2% MPBST at room temperature (20°C).

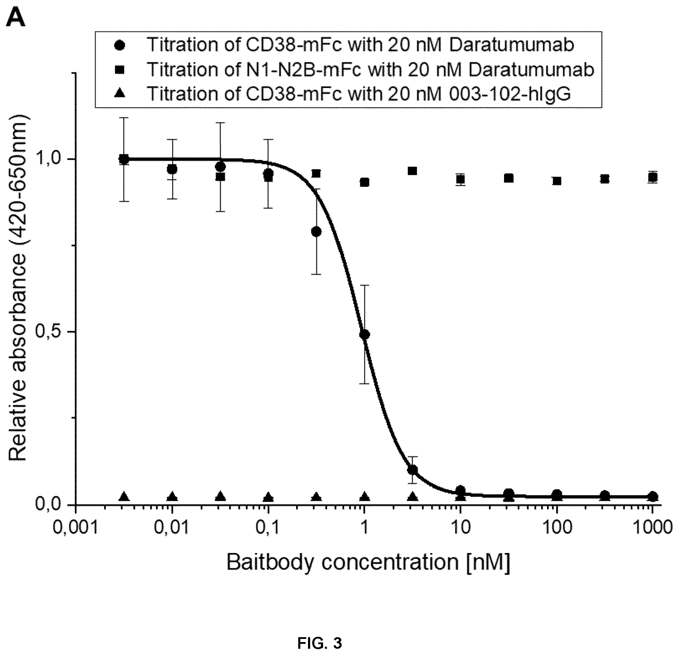

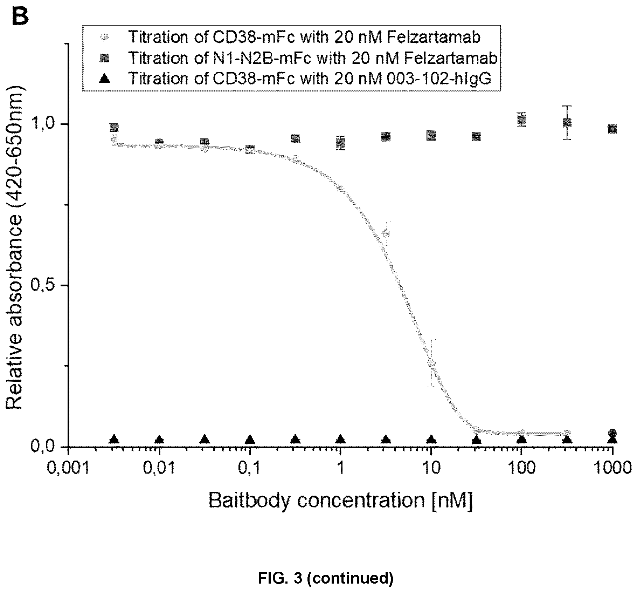

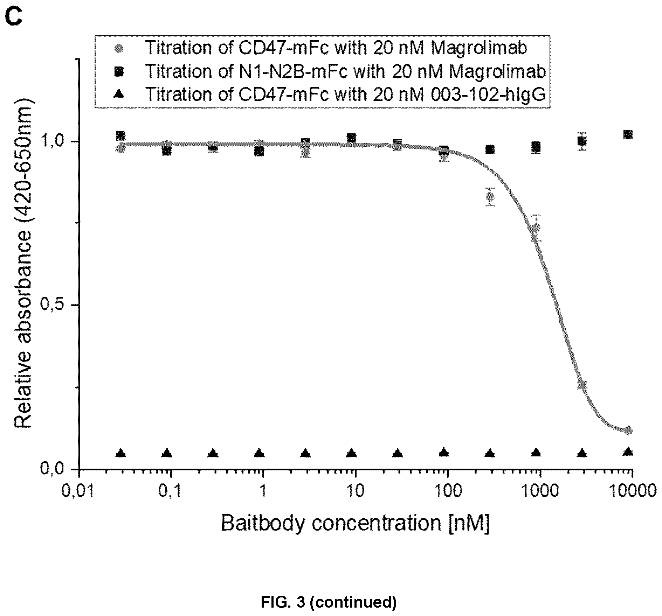

**Figure 3** - Results of inhibition ELISA with the CD38-mFc and CD47-mFc baitbodies. The relative absorbance (420-650nm) correlating the binding intensity was plotted against the baitbody concentration in nM. 20 nM of the therapeutic antibodies daratumumab (A), felzartamab (B) and magrolimab (C) were pre-incubated with the corresponding baitbody CD38-mFc (A and B) and CD47-mFc (C). The bound therapeutic antibodies to immobilized baitbody were detected with a goat anti-human IgG-peroxidase (A0170). The N1-N2B-mFc baitbody and the antibody 003-102-hIgG were used as controls for the binding specificity. Dots in red were masked to allow a fit to be calculated. All steps were carried out in 2% MPBST at room temperature (20°C).

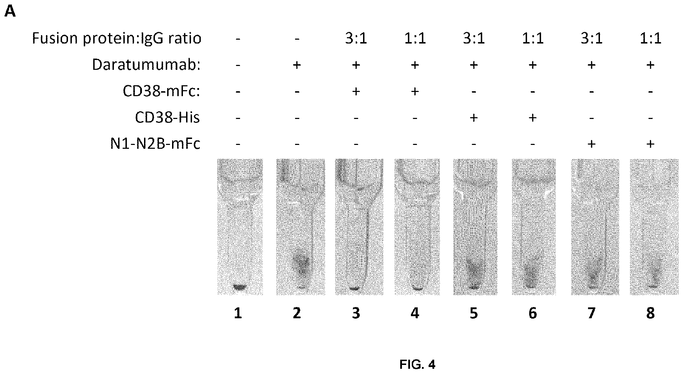

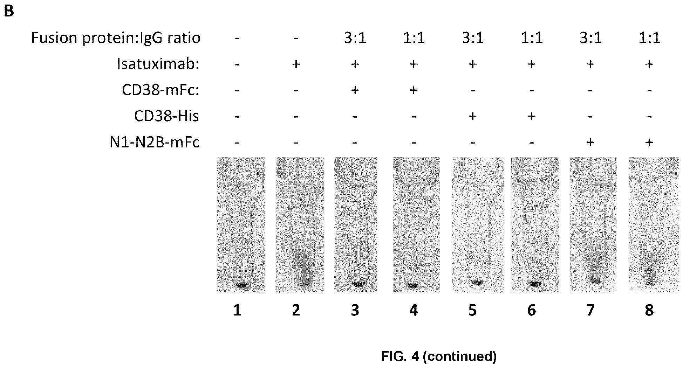

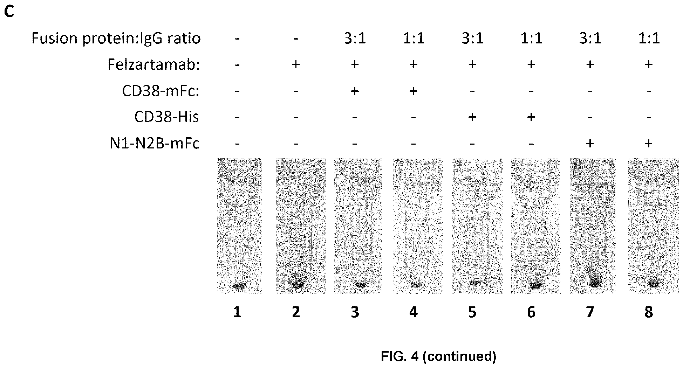

**Figure 4** - Results of indirect antiglobulin tests using plasma spiked with daratumumab and felzartamab. A: The indirect antiglobulin tests (IATs) were carried out with human plasma (A1) with the addition of 100 nM daratumumab (A2-8). CD38-mFc baitbodies were added in a 3:1 (A3) and 1:1 ratios (A4), CD38-His in a 3:1 (A5) and 1:1 ratio (A6) or N1-N2B-mFc in 3:1 (A7) and 1:1 ratios (A8). B: Human plasma (B1) was also spiked with 100 nM isatuximab (B2-8). Fusion proteins CD38-mFc were added in a 3:1 (B3) and 1:1 ratios (B4), CD38-His in a 3:1 (B5) and 1:1 ratio (B6) or N1-N2B-mFc in 3:1 (B7) and 1:1 ratios (B8). C: Human plasma (C1) was also spiked with 100 nM felzartamab (C2-8). Fusion proteins CD38-mFc were added in a 3:1 (C3) and 1:1 ratios (C4), CD38-His in a 3:1 (C5) and 1:1 ratio (C6) or N1-N2B-mFc in 3:1 (C7) and 1:1 ratios (C8). The erythrocytes used were ID Dia cells I, cells II and cells III. The IAT images presented here were conducted using ID Dia cells I. After plasma and erythrocytes were mixed on ID card test columns, the cards were incubated for 15 min at 37°C and centrifuged for 10 min at 910 rpm. Afterwards the agglutination reaction was documented via photography.

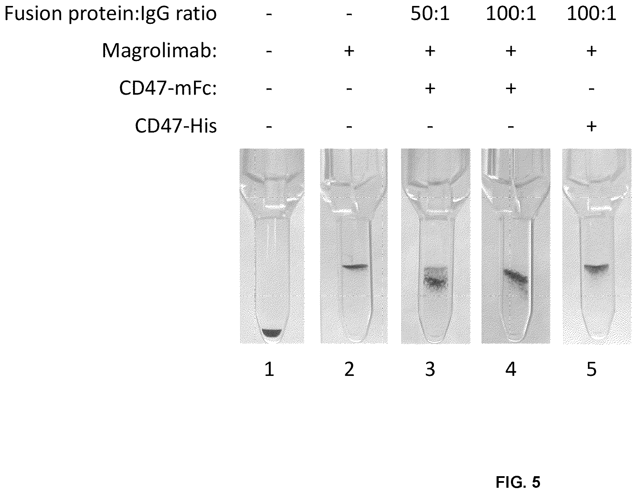

**Figure 5** - Results of indirect antiglobulin tests using plasma spiked with magrolimab. The indirect antiglobulin tests were carried out with human plasma (1) and with the addition of 100 nM magrolimab (2-5). CD47-mFc baitbodies were added in a 50:1 (3) and 100:1 ratio (4) or CD47-His added in a 100:1 ratio (5). The erythrocytes used were ID Dia cells I, cells II and cells III. The IAT images presented here were conducted using ID Dia cells I. After plasma and erythrocytes were mixed on ID card test columns, the cards were incubated for 15 min at 37°C and centrifuged for 10 min at 910 rpm. Afterwards the agglutination reaction was documented via photography.

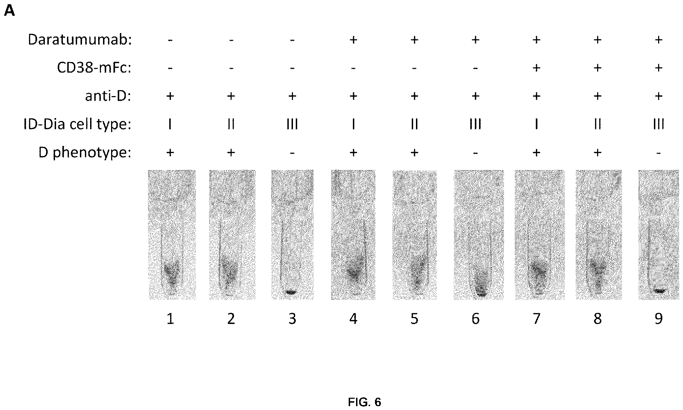

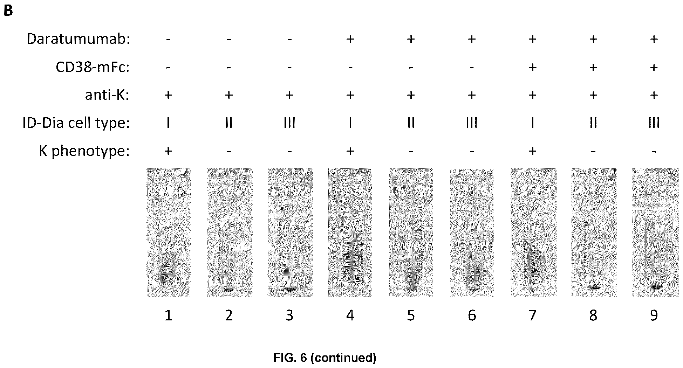

**Figure 6** - Results of antibody screening indirect antiglobulin tests using serum spiked with anti-D and anti-K alloantibodies and Daratumumab. The indirect antiglobulin tests were carried out with test human serum with anti-D (A) and anti-K (B) alloantibodies and ID-DiaCells I, cells II and cells III as test erythrocytes (A1-3, B1-3). ID-DiaCell phenotypes are listed table 2. 500 nM Daratumumab was added (A4-6, B4-6) and competition was carried out with CD38-mFc in a 3:1 ratio (A7-9, B7-9). After plasma and erythrocytes were mixed on the ID card test columns, the cards were incubated for 15 min at 37°C and centrifuged for 10 min at 910 rpm. Afterwards the agglutination reaction was documented via photography.

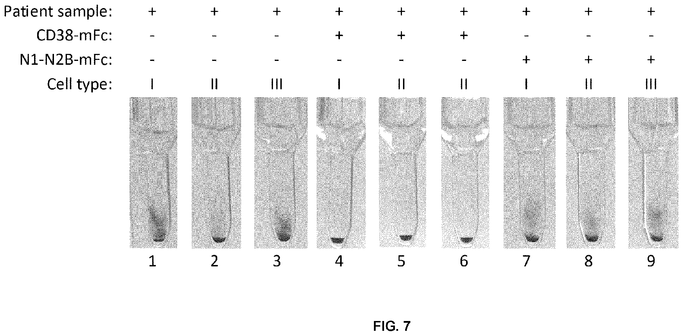

**Figure 7** - Results of indirect antiglobulin tests using daratumumab patient samples. The indirect antiglobulin tests were carried out with serum samples from patient 1, who was treated with daratumumab (1-3). The erythrocytes used were ID Dia cells I II III. 4160 nM CD38-mFc (4-6) or N1-N2B-mFc (7-9) was added to the serum. After plasma and erythrocytes were mixed on ID card test columns, the cards were incubated for 15 min at 37°C and centrifuged for 10 min at 910 rpm. Afterwards the agglutination reaction was documented via photography. Patient 1 is shown as an example.

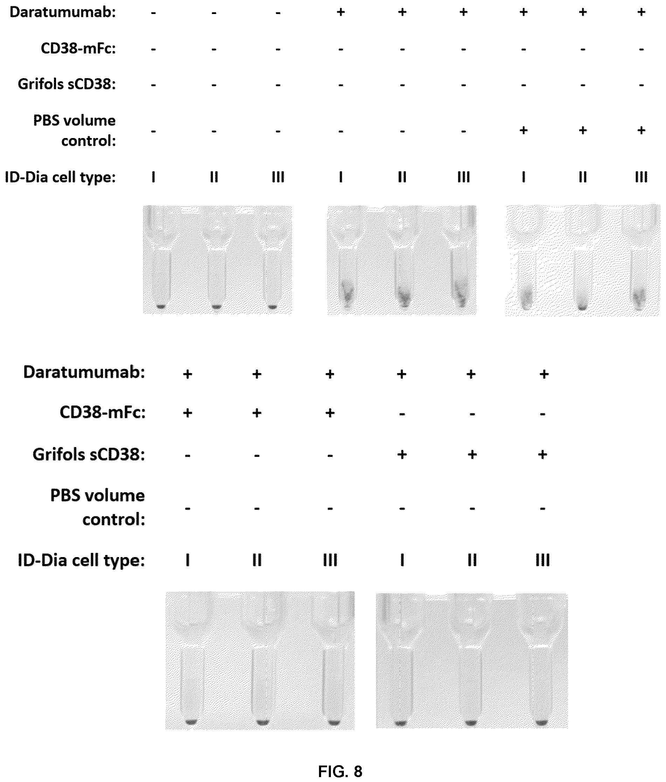

**Figure 8** -Results of indirect antiglobulin tests using serum spiked with daratumumab. The indirect antiglobulin tests were carried out with donor human plasma and ID Dia cells I, cells II and cells III as test erythrocytes. ID Dia cell phenotypes are listed table 2. The plasma was spiked with 0.5 mg/mL daratumumab, and competition was carried out with CD38-mFc and Grifols sCD38. After plasma and erythrocytes were mixed on the ID card test columns, the cards were incubated for 15 min at 37°C and centrifuged for 10 min at 910 rpm. Afterwards the agglutination reaction was documented via photography.

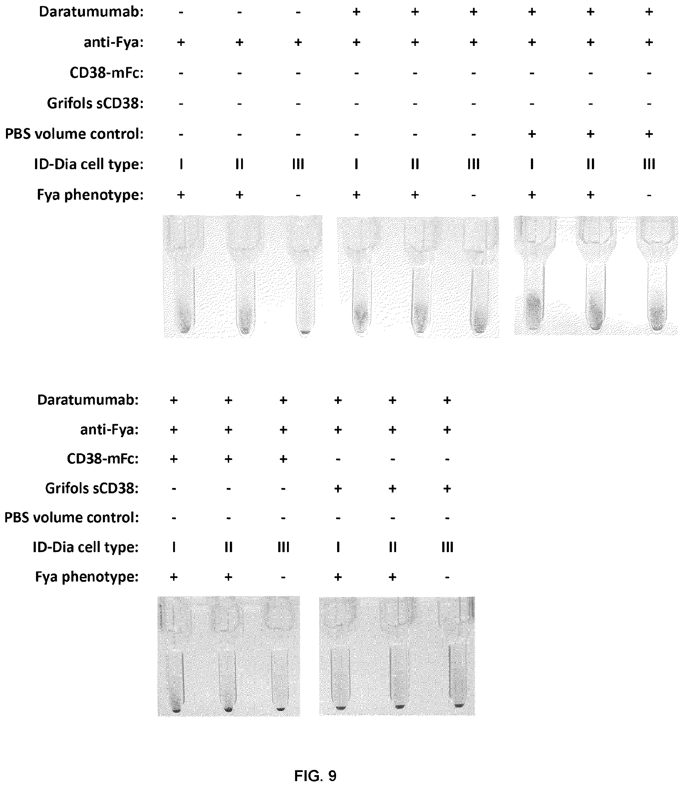

**Figure 9** - Results of indirect antiglobulin tests using serum spiked with anti-Fya alloantibodies and daratumumab. The indirect antiglobulin tests were carried out with test human plasma with anti-Fya alloantibodies and ID Dia cells I, cells II and cells III as test erythrocytes. ID Dia cell phenotypes are listed in table 2. The plasma was spiked with 0.5 mg/mL daratumumab, and competition was carried out with CD38-mFc and Grifols sCD38. After plasma and erythrocytes were mixed on the ID card test columns, the cards were incubated for 15 min at 37°C and centrifuged for 10 min at 910 rpm. Afterwards the agglutination reaction was documented via photography.

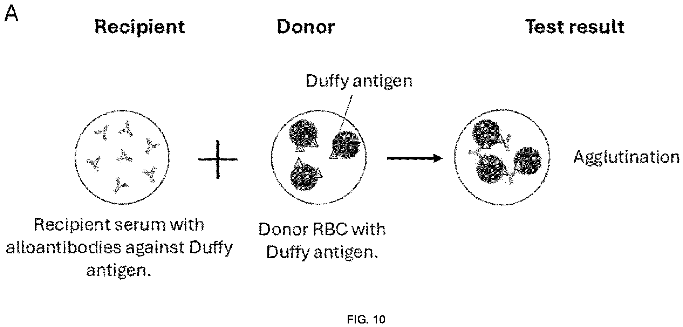

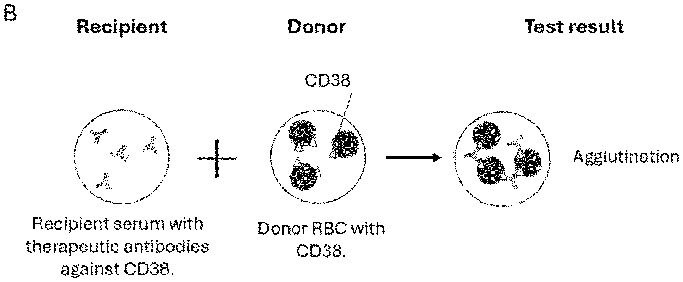

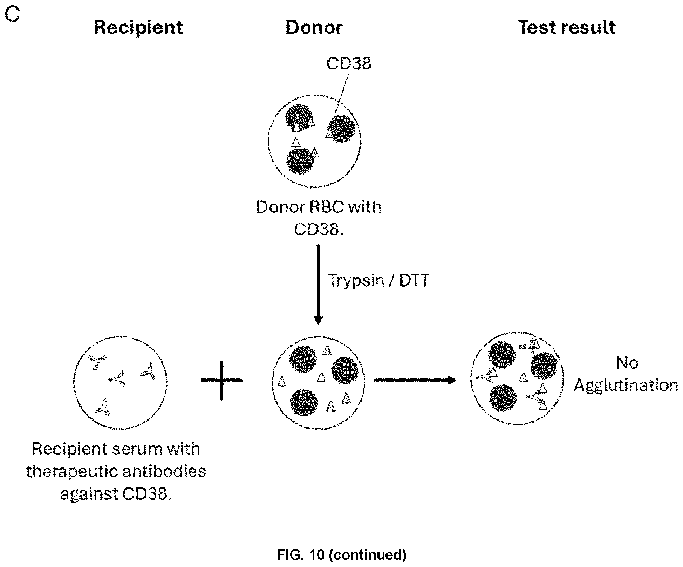

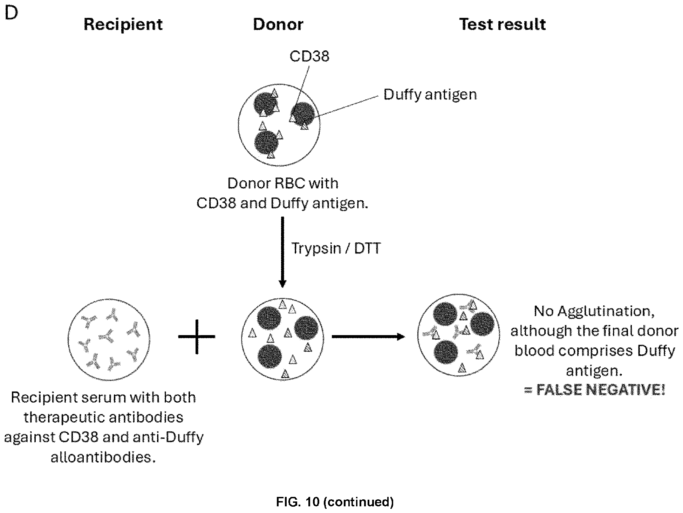

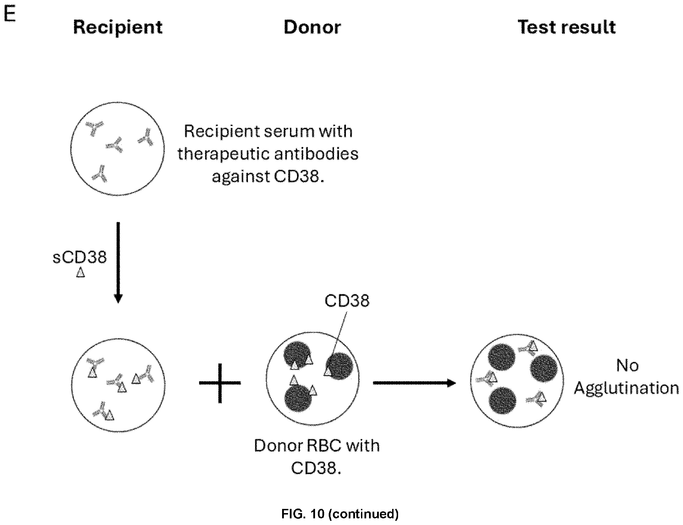

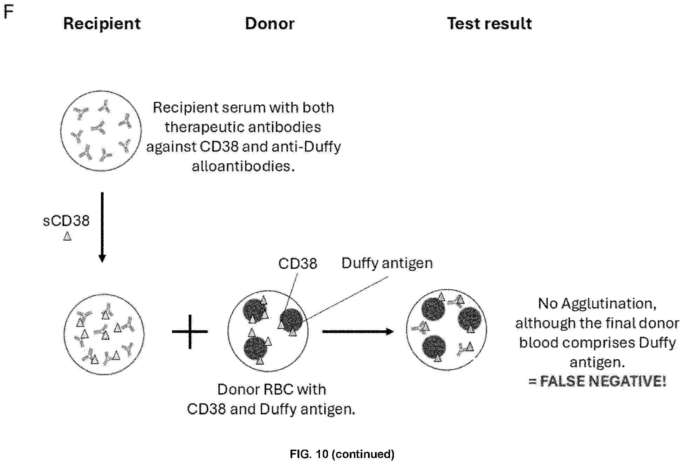

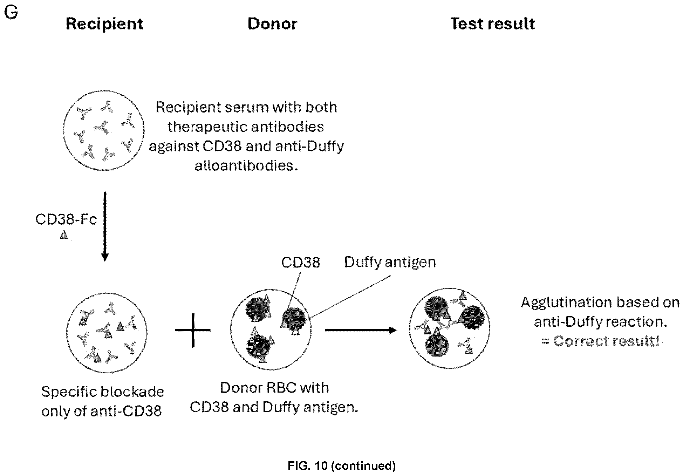

**Figure 10** - Alloantibodies against Duffy antigens in donor blood can cause agglutination and potentially lead to dangerous hemolysis in recipients receiving such blood (Figure 12A). Additionally, patients undergoing anti-cancer treatments with therapeutic antibodies targeting CD38 may experience agglutination reactions to donor blood containing the CD38 antigen on red blood cells (RBCs), resulting in false-positive indirect antiglobulin tests (!ATs) (Figure 12B). Various mitigation strategies have been proposed to prevent interference from therapeutic anti-CD38 antibodies. The most common approach is to treat RBCs with dithiothreitol (DTT) to denature protein targets like CD38. Alternatively, proteases such as trypsin can cleave the extracellular domain of CD38, which has been shown to prevent agglutination caused by anti-CD38 antibodies (as illustrated in Figure 12C). While this is a cost-effective strategy, denaturing reagents or proteases can interfere with other surface proteins that constitute the majority of RBC antigens, such as Duffy antigens (Figure 12D). Another solution involves neutralizing the therapeutic antibodies with anti-idiotypic antibodies or soluble antigens (Figure 12E). These can be added to the patient's serum before the IAT and have been shown to effectively prevent false positive agglutination. However, anti-idiotypic antibodies are very expensive, and soluble CD38 can cross-react with alloantibodies, potentially masking reactions like anti-Duffy (Figure 12F). The fusion protein described in the present disclosure specifically binds to therapeutic anti-CD38 antibodies without masking other alloantibodies in the recipient's serum, thereby ensuring accurate detection of agglutination caused by anti-Duffy antibodies. This significantly reduces health risks for the blood recipient. The situation for CD47 is comparable.

Examples

**Example 1: Baitbody characterization**

Prior to the application in blood compatibility testing, the generated baitbodies were characterized in several assays. The functionality of the constructs and binding of the target therapeutic anti-CD38 and anti-CD47 monoclonal antibodies was tested. Furthermore, the affinity based on the half maximal effective concentration (EC50) as well as the specificity was assessed. The approximate amount baitbody necessary for the inhibition of therapeutic antibody binding was evaluated.

**Example 1.1: Evaluation of binding, affinity and specificity**

The binding of the baitbody constructs to the therapeutic antibodies was evaluated in a titration ELISA. The therapeutic antibodies were titrated on immobilized CD38-mFc and CD47-mFc to estimate the binding affinity. Additionally, the N1-N2B-mFc baitbody was used to test the binding specificity of daratumumab, isatuximab and felzartamab to CD38-mFc and magrolimab to CD47-mFc. The specificity of the baitbodies was also verified with the antibody 003-102-hIgG during the titration of the baitbodies on immobilized antibodies. The results showed that both daratumumab and Felzartamab exhibited specific binding to CD38-mFc (figure 1). The EC50-values of the three antibodies were comparable with 0.12 nM for daratumumab, 0.18 nM for isatuximab and 0.32 nM for felzartamab.

The characterization of CD47-mFc was performed with magrolimab (figure 2). Magrolimab was shown to bind specifically to CD47-mFc as no significant binding to N1-N2B-mFc could be detected. The EC50-value was 0.21 nM and in a similar range as the values determined for the binding of CD38-mFc and the anti-CD38 antibodies tested.

**Example 1.2: Evaluation of inhibition potential**

For further characterization and to assess the amount of baitbody needed to fully inhibit the binding of the therapeutic antibodies to an immobilized target, inhibition ELISAs were performed with daratumumab (figure 3A), felzartamab (figure 3B) and magrolimab (figure 3C). CD38-mFc was able to inhibit the binding of both tested anti-CD38 antibodies, while daratumumab was inhibited better than felzartamab. CD47-mFc was able to inhibit magrolimab but required a significantly higher amount of baitbody.

**Example 2: Baitbody application in serological assays**

After the characterization, the baitbody constructs were tested in the indirect antiglobulin test (IAT). This assay is a crucial part of pre-transfusion testing and involves mixing recipient serum or plasma with test erythrocytes to check for agglutination reactions caused by alloantibodies against RBC antigens. The presence of anti-CD38 and anti-CD47 antibodies in the recipient sample causes false positive agglutination, hindering the tests. The baitbodies were evaluated in the ability to neutralize the therapeutic antibodies daratumumab, isatuximab felzartamab and magrolimab and thus to mitigate this false positive agglutination, while allowing alloantibodies to be detected. This was investigated with samples where therapeutic antibodies were added prior to the tests and with samples of patients treated with daratumumab. Additionally, it was examined if preventing the false positive agglutination with the CD38-mFc baitbody would interfere with the detection of alloantibodies against the D antigen of the Rheusus blood group system and K antigen of the Kell blood group system in serum. The level of agglutination was estimated on a scale of 0 to 4+ (++++) using the ID card interpretation guide according to the manufacturer guide.

Example 2.1: Baitbody competition in spiked samples. To test if the baitbody constructs can prevent false positive agglutination, donor plasma without any relevant RBC alloantibodies or therapeutic antibodies functioned as a basis and the target therapeutic antibodies were added. Three ID cell types with different RBC antigen variations were utilized as test erythrocytes. The baitbody construct CD38-mFc was tested with three different anti-CD38 antibodies, daratumumab, isatuximab and felzartamab. While all three antibodies caused false positive IATs, the agglutination reaction levels varied. Daratumumab caused the strongest false positive agglutination reactions up to 2+ (++) with all three ID cell types at a concentration of 100 nM (figure 10A). The baitbody was added in a three-fold molar excess as well as the molar equivalent. The same was done with soluble CD38-His and N1- N2B-mFc (a control baitbody with the same Fc-domain). The addition of CD38-mFc in three-fold excess resulted in complete mitigation, while the interference persisted at the molar equivalent. Soluble CD38-His did not prevent the agglutination at these ratios. Isatuximab caused a similarly strong agglutination reaction in the IAT of ID cell type I, while the agglutination reactions of the remaining cell types were very slight at 100 nM. The same ratios and fusion proteins were tested for neutralization. Despite the strength of the reaction, the agglutination was prevented by both CD38-mFc and CD38-His at a molar equivalent (figure 10B). At the same concentration felzartamab caused a weaker reaction but resulted in macroscopically positive IATs for all three ID cell types (supplementary figure 20). In this case, soluble CD38-His was able to mitigate the interference at a three-fold excess, while CD38-mFc was able to prevent any agglutination at a 1:1 ratio (figure 10C). The control baitbody N1-N2B-mFc did not have any effects.

The anti-CD47antibody magrolimab caused a very strong agglutination reaction rated at 4+ (++++), which was evident at the test concentration of 100 nM. The false positive agglutination was not prevented either by competition with the CD47-mFc baitbody nor by CD47-His. The baitbody was tested in 50-fold and 100-fold excess and the soluble protein at a 100-fold excess. These high excesses of baitbody caused a slight reduction of the false positive agglutination reaction but failed to prevent it.

**Example 2.2: Baitbody competition in spiked samples with alloantibodies**

A crucial prerequisite of any mitigation strategy is not to interfere with the antigens central to the blood group systems. To assess whether the addition of baitbodies hinders the detection of alloantibodies, antibody screening IATs were conducted using test serum containing alloantibodies against the D antigen of the Rheusus blood group system and K antigen of the Kell blood group system. Of the three test erythrocytes, the ID-Dia Cell type I and type II had a positive D phenotype and the ID-Dia cell type I was K positive. The presence of the antigen on the test erythrocytes and the corresponding alloantibodies caused agglutination in column agglutination tests, while the tests with test erythrocytes with a negative phenotype had no agglutination. Daratumumab was added to the serum in a concentration of 500 nM and caused false positive agglutination in all the column agglutination tests regardless of phenotype of the test erythrocytes, masking the presence of alloantibodies. Given the successful complete mitigation observed with a three-fold excess of CD38-mFc, as detailed above, the same ratio was applied in this experiment. The addition of the baitbody construct to the spiked serum prevented the false positive agglutination and revealed the same agglutination pattern for the anti-D (figure 6A) and anti-K (figure 6B) alloantibodies as prior to the daratumumab addition.

**Example 2.3: Baitbody competition in patient samples**

After testing the baitbody competition of daratumumab in spiked plasma and serum samples, the effectiveness of CD38-mFc was investigated in patient samples. For the competition, CD38-mFc was added to the samples at a concentration of 4160 nM. To test cross reactivity to murine Fc-domain, the same concentration of control baitbody N1-N2B-mFc was used.. The IAT results of patient one showed a complete prevention of agglutination reaction after the initial addition of CD38-mFc, while the addition of the control baitbody did not affect the false positive agglutination level (figure 7).

The competition with CD38-mFc was performed in eight patient samples (table 1). The agglutination level caused by daratumumab in patient samples was relatively consistent and was estimated as +1 or +2. The agglutination reaction was mitigated completely at the initial baitbody concentration of 4160 nM (120 μg) in the patient samples one, two, five, six, seven and eight. At the same initial concentration, the IATs of patient three remained macroscopically positive for the ID cells I and III. The IAT of ID cell II was negative. After the CD38-mFc concentration was increased to 5200 nM (240nμg), agglutination could no longer be observed. Similarly, the IATs of patient four with ID cells I and II showed agglutination at a level of +1, while the IAT with ID cell III showed no macroscopic reaction. An increase of the CD38-mFc concentration did result in a decrease in agglutination, with the IAT of ID cell I showing no macroscopic agglutination. This agglutination after the initial competition may however be the result of RBC alloantibodies previously masked by the false positive agglutination.

Table 1: Results of indirect antiglobulin tests with patient samples (1-9) and competition with the CD38-mFc baitbody

| Patient | CD38-mFc concentration [nM] | Agglutination (0 to ++++) | | |
|---|---|---|---|---|
| | | Cell I | Cell II | Cell III |
| 1 | - | + | + | + |
| | 4160 nM | 0 | 0 | 0 |
| 2 | - | ++ | ++ | ++ |
| | 4160 nM | 0 | 0 | 0 |
| 3 | - | + | + | + |
| | 4160 nM | ± | 0 | ± |
| | 5200 nM | 0 | 0 | 0 |
| 4 | - | ++ | ++ | + |
| | 4160 nM | + | + | 0 |
| | 5200 nM | 0 | ± | 0 |
| 5 | - | + | + | + |
| | 4160 nM | 0 | 0 | 0 |
| 6 | - | ++ | + | ++ |
| | 4160 nM | 0 | 0 | 0 |
| 7 | - | ++ | ++ | + |
| | 4160 nM | 0 | 0 | 0 |
| 8 | - | + | + | ++ |
| | 4160 nM | 0 | 0 | 0 |
| 9 | - | ++ | + | + |
| | 4160 nM | 0 | 0 | 0 |

Example 3: Head-to-head comparison of the baitbody CD38-mFc according to the disclosure and prior art sCD38

In order to compare the features of the baitbody construct CD38-mFc according to the disclosure and a prior art soluble CD38 (sCD38) construct (Grifols Diagnostic Solutions) a head-to-head comparison test was performed.

The sCD38 of Grifols is described in the documentation as "Antibodies against Duffy antigens may react weaker or negatively after Grifols sCD38 treatment." (hftps://www.diagnostic.grifols.com/documents/5952084/5955218/Brochure Grifols sCD381.pdf/ee5c7720-e332-646a-7e40-74f660bd3780?t=1705422448428).

In order to test the superiority of the baitbody construct CD38-mFc according to the disclosure vs. the prior art sCD38 provided by Grifols, donor plasma with a daratumumab concentration of 0.5 mg/mL was prepared. The protocol for Grifols sCD38 instructed the addition of 2 μl of the reagent (30 mg/mL) per 25 μl of plasma sample.

The baitbody construct was added with 2.5 μl per 25 μl from a stock solution of 16.7 mg/mL. After the addition of Grifols sCD38 and the baitbody, the spiked plasma was incubated at RT for 15 min while the test columns were prepared. 25 μl plasma and 50 μl test erythrocytes of the three-cell panel (ID-DiaCell I-II-III, a commercially available standardized test system from Bio-Rad) were then mixed on the ID card test columns and the cards were incubated for 15 min at 37°C followed by centrifugation for 10 min at 910 rpm. ID Dia cell phenotypes are listed in table 2 below, relevant for the test were only the Duffy antigens.

**Table 2: Biorad ID-DiaCell I-II-III antigen list (Set IP-IIP-IIIP: 45194.50.x, expires: 2024/08/19) nt: not tested**

| | Rh-Hr | | | | | | Kell | | | | | | Duffy | | Kidd | |
|---|---|---|---|---|---|---|---|---|---|---|---|---|---|---|---|---|
| Cell | D | C | c | E | e | C | K | k | $Kp^a$ | $Kp^b$ | $Js^a$ | $Js^b$ | $F_y{}^a$ | $F_y{}^b$ | $Jk^a$ | $Jk^b$ |
| I | + | + | 0 | 0 | + | + | + | + | 0 | + | nt | nt | + | 0 | + | 0 |
| II | + | 0 | + | + | 0 | 0 | 0 | + | 0 | + | nt | nt | + | + | + | + |
| III | 0 | 0 | 0 | + | + | 0 | 0 | + | 0 | + | nt | nt | 0 | + | 0 | + |

| | Lewis | | P | MN | | | | Lutheran | | Xg |
|---|---|---|---|---|---|---|---|---|---|---|
| Cell | $Le^a$ | $Le^b$ | $P_1$ | M | N | S | s | $Lu^a$ | $Lu^b$ | $Xg^a$ |
| I | 0 | + | 0 | 0 | + | 0 | + | + | + | + |
| II | 0 | + | + | + | 0 | + | 0 | 0 | + | + |
| III | + | 0 | + | + | + | + | + | 0 | + | + |

Both constructs mitigated the agglutination caused by daratumumab (shown in figure 10).

To assess whether the addition of CD38-mFc or Grifols sCD38 hinders the detection of alloantibodies, antibody screening IATs were conducted using test plasma containing alloantibodies against the Fya antigen of the Duffy blood group system. The Fya phenotypes of the three-cell panel (ID-DiaCell I-II-III, Bio-Rad) shown in figure 11. The presence of the antigen on the test erythrocytes and the corresponding alloantibodies in the serum induced agglutination, resulting in a specific pattern.

While the ID-DiaCells I and II with the Fya antigen remained positive after competition with the baitbody construct, all the test columns showed no agglutination reaction after the addition of Grifols sCD38, which did not allow the Fya alloantibodies to be detected.

Example 4: Baitbody competition **in** solid phase testing method

The competition with the CD38 baitbody constructs were also tested in an antibody identification 14 cell panel with the solid phase Capture-R® method on the automated NEO® system. The cells with unique antigen compositions were coated on the surface of each well of the panel with the addition of a positive and negative control as the 15th and 16th cells. The detection of antibodies was achieved in the solid phase method by the antibodies in the sample binding to the antigens on the coated cells and then being bound by anti-human IgG coated on indicator cells. Through a centrifugation step, unbound indicator cells are pooled in the center of the well, while bound indicator cells remain spread out. The antibody identification panel was carried out with a sample of test human serum on the NEO® system. 500 nM Daratumumab was added, and competition was carried out with CD38-mFc in a 3:1 ratio. Each well was documented by the camera reader and analyzed by the NEO® system. The reaction strengths were evaluated and presented by the system as a grade (0, 1, 2, 3, 4). The reaction strength values are shown in table 3. After the completion of each test, the results were reviewed and compared to a visual assessment of the plates, which resulted in the final grade.

The donor sample showed no agglutination for any of the 14-cell-panel, while the addition of Daratumumab resulted in false positive reaction strength values for the entire panel as well as multiple cells being graded 1. After the competition with CD38-mFc the false positive reaction strengths were reduced significantly enough to allow all the cells of the panel to be graded 0, thus preventing the false positive agglutination.

Table 3: Results of antibody identification Capture-R® Ready-ID® panel using serum spiked with Daratumumab.

| Cell | Donor | | Daratumumab | | Daratumumab +CD38-mFc | |
|---|---|---|---|---|---|---|
| | Strength | Grade | Strength | Grade | Strength | Grade |
| 1 | 0.00 | 0 | 24.7 | + | 1.20 | 0 |
| 2 | 0.00 | 0 | 24.5 | + | 1.60 | 0 |
| 3 | 0.00 | 0 | 10.7 | + | 0.00 | 0 |
| 4 | 0.00 | 0 | 9.10 | - | 0.00 | 0 |
| 5 | 0.00 | 0 | 28.2 | + | 1.40 | 0 |
| 6 | 0.00 | 0 | 16.8 | + | 0.00 | 0 |
| 7 | 0.00 | 0 | 12.1 | + | 0.00 | 0 |
| 8 | 0.00 | 0 | 20.6 | + | 3.20 | 0 |
| 9 | 0.00 | 0 | 21.5 | + | 0.00 | 0 |
| 10 | 0.00 | 0 | 13.7 | - | 0.00 | 0 |
| 11 | 0.00 | 0 | 36.4 | + | 2.10 | 0 |
| 12 | 0.00 | 0 | 22.7 | + | 0.70 | 0 |
| 13 | 0.00 | 0 | 38.2 | + | 1.20 | 0 |
| 14 | 0.00 | 0 | 10.6 | - | 0.10 | 0 |
| PC | 99.9 | ++++ | 94.3 | ++++ | 99.9 | ++++ |
| NC | 0.00 | 0 | 0.00 | - | 0.00 | 0 |

## Claims

**1.** A fusion protein for binding to an agglutinating antibody, comprising

an extracellular domain of CD38 and/or CD47, as well as fragment thereof,
optionally, a linker region,
a hinge-region,
and an Fc-fragment.

**2.** The fusion protein according to claim 1, wherein the sequence of the extracellular domain of CD38, and/or a fragment thereof, is selected from SEQ ID. No. 1 or 2 and the extracellular domain of CD47, and/or a fragment thereof, is selected from SEQ ID. No. 3.

**3.** The fusion protein according to claims 1 or 2, wherein the linker region comprises the sequence according to SEQ ID. No. 4 or 5, as well as variants thereof with up to one amino acid difference in form of a point-mutation, deletion and/or insertion as compared to SEQ ID. No. 4 or 5.

**4.** The fusion protein according to one or more of claims 1 to 3, wherein the hinge-region comprises the sequence according to SEQ ID. No. 6 to 9, as well as variants thereof with up to two amino acids difference in form of a point-mutation, deletion and/or insertion as compared to SEQ ID. No. 6 to 9.

**5.** The fusion protein according to one or more of claims 1 to 4, wherein the sequence of the Fc fragment has at least 98% sequence identity to a sequence selected from the group consisting of SEQ ID. No. 10 to 18.

**6.** The fusion protein according to one or more of the previous claims, wherein the sequence of the fusion protein has at least 97% sequence identity to a sequence selected from the group consisting of SEQ ID. No. 19 to 342.

**8.** The fusion protein according to any of the previous claims, having at least one of the following characteristics:

- it has an EC50-value of 0.35 nM or less;
- it prevents agglutination mediated by the cross-reaction of an anti-CD38 with CD38 and/or anti-CD47-antibody with CD47 in a blood-sample when added with at least an equimolar ratio as compared to the agglutinating antibody;
- it prevents agglutination mediated by the cross-reaction of an anti-CD38 with CD38 and/or anti-CD47-antibody with CD47 in a blood-sample when added with at most 45-fold molar excess as compared to the agglutinating antibody;
- it prevents agglutination mediated by the cross-reaction of an anti-CD38 with CD38 and/or anti-CD47-antibody with CD47 in a blood-sample when present in the blood-sample at a concentration of 100,000 nM or less.

**9.** An analytical, diagnostic or pharmaceutical composition comprising the fusion protein according to any of claims 1 to 8, further comprising at least one compound selected from the group consisting of a buffer, a stabilizer, a diluent, a filler, a binder, a disintegrant, a glidant, a preservative, a solvent, a surfactant, as well as any combinations thereof.

**10.** Use of a fusion protein according to any of claims 1 to 8, or the composition according to claim 9, for the prevention of agglutination mediated by the cross-reaction of an anti-CD38 with CD38 and/or anti-CD47-antibody with CD47 in a blood-sample.

**11.** The use according to claim 10, wherein the blood-sample of the recipient and/or donor cannot be excluded of being positive for the presence of antibodies against at least one of the antigens selected from the group consisting of $Fy^a$ (Duffy a), $Fy^b$ (Duffy b), $Fy^3$, $Fy^4$, $Fy^5$, $Fy^6$, and $Fy^x$, as well as combinations thereof.

**12.** The use according to claims 10 or 11, wherein the blood-sample of the recipient and/or donor cannot be excluded of being positive for the presence of antibodies against CD38 and/or CD47.

**13.** Fusion protein according to any of claims 1 to 8 for use in a method of treating a patient, or the composition according to claim 9 for use in a method of treating a patient, wherein the treatment comprises

- taking a sample volume from the patient, such as blood, plasma or serum,
- testing the sample volume for compatibility with a donor sample, the donor sample comprising red blood cells, wherein the testing comprises contacting the fusion protein or the composition with the sample volume or the donor sample, and combining the sample volume with the donor sample,
- administering a blood donation of the donor to the patient in case of compatibility,

wherein said patient or said donor cannot be excluded of having antibodies against at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof; and/or wherein said patient or said donor is exhibiting expression of at least one of the antigens selected from $Fy^a$ (Duffy a), $Fy^b$ (Duffy b), $Fy^3$, $Fy^4$, $Fy^5$, $Fy^6$, and $Fy^x$, as well as combinations thereof.

**14.** A method for neutralizing or blocking the binding of an anti-CD38 and/or an anti-CD47 antibody in a sample, comprising:

providing a volume of the sample, consisting of blood, plasma, and serum, containing the anti-CD38 and/or CD47 antibody; and

incubating the sample with the fusion protein according to any of claims 1 to 8 or a volume of the composition according to claim 9, comprising an amount of the fusion protein sufficient to specifically neutralize the anti-CD38 and/or CD47 antibody in the sample.

**15.** A kit for bio-monitoring research and diagnostic assays, comprising the fusion protein according to any of claims 1 to 8, or the composition according to claim 9, a plate and reagents for identifying the presence of antibodies.

Daratumumab titration on CD38-mFc (EC50 = 0.12 nM)
Daratumumab titration on N1-N2B-mFc
Isatuximab titration on CD38-mFc (EC50 = 0.18 nM)
Isatuximab titration on N1-N2B-mFc
Felzartamab titration on CD38-mFc (EC50 = 0.32 nM)
Felzartamab titration on CD38-mFc
Relative absorbance (420-650 nm)
1,0
0,5
0,0
0,001
0,01
0,1
1
10
100
IgG concentration [nM]


FIG. 1

Magrolimab titration on CD47-mFc (EC50 = 0.21 nM)
Magrolimab titration on N1-N2B-mFc
Relative absorbance (420-650nm)
1,0
0,5
0,0
0,001
0,01
0,1
1
10
100
IgG concentration [nM]

FIG. 2

A
Titration of CD38-mFc with 20 nM Daratumumab
Titration of N1-N2B-mFc with 20 nM Daratumumab
Titration of CD38-mFc with 20 nM 003-102-hIgG
Relative absorbance (420-650nm)
1,0
0,5
0,0
0,001
0,01
0,1
1
10
100
1000
Baitbody concentration [nM]

FIG. 3

B
Titration of CD38-mFc with 20 nM Felzartamab
Titration of N1-N2B-mFc with 20 nM Felzartamab
Titration of CD38-mFc with 20 nM 003-102-hIgG
Relative absorbance (420-650nm)
1,0
0,5
0,0
0,001
0,01
0,1
1
10
100
1000
Baitbody concentration [nM]

FIG. 3 (continued)

C
Titration of CD47-mFc with 20 nM Magrolimab
Titration of N1-N2B-mFc with 20 nM Magrolimab
Titration of CD47-mFc with 20 nM 003-102-hIgG
Relative absorbance (420-650nm)
1,0
0,5
0,0
0,01
0,1
1
10
100
1000
10000
Baitbody concentration [nM]

FIG. 3 (continued)

**A**

| Fusion protein:IgG ratio | - | - | 3:1 | 1:1 | 3:1 | 1:1 | 3:1 | 1:1 |
|---|---|---|---|---|---|---|---|---|
| Daratumumab: | - | + | + | + | + | + | + | + |
| CD38-mFc: | - | - | + | + | - | - | - | - |
| CD38-His | - | - | - | - | + | + | - | - |
| N1-N2B-mFc | - | - | - | - | - | - | + | + |
| | **1** | **2** | **3** | **4** | **5** | **6** | **7** | **8** |

FIG. 4

**B**

| Fusion protein:IgG ratio | - | - | 3:1 | 1:1 | 3:1 | 1:1 | 3:1 | 1:1 |
|---|---|---|---|---|---|---|---|---|
| Isatuximab: | - | + | + | + | + | + | + | + |
| CD38-mFc: | - | - | + | + | - | - | - | - |
| CD38-His | - | - | - | - | + | + | - | - |
| N1-N2B-mFc | - | - | - | - | - | - | + | + |
| | 1 | 2 | 3 | 4 | 5 | 6 | 7 | 8 |

FIG. 4 (continued)

C

| | | | | | | | | |
|---|---|---|---|---|---|---|---|---|
| Fusion protein:IgG ratio | - | - | 3:1 | 1:1 | 3:1 | 1:1 | 3:1 | 1:1 |
| Felzartamab: | - | + | + | + | + | + | + | + |
| CD38-mFc: | - | - | + | + | - | - | - | - |
| CD38-His | - | - | - | - | + | + | - | - |
| N1-N2B-mFc | - | - | - | - | - | - | + | + |
| | 1 | 2 | 3 | 4 | 5 | 6 | 7 | 8 |

FIG. 4 (continued)

| | | | | | |
|---|---|---|---|---|---|
| Fusion protein:IgG ratio | - | - | 50:1 | 100:1 | 100:1 |
| Magrolimab: | - | + | + | + | + |
| CD47-mFc: | - | - | + | + | - |
| CD47-His | - | - | - | - | + |

1
2
3
4
5

FIG. 5

**A**

| | | | | | | | | | |
|---|---|---|---|---|---|---|---|---|---|
| Daratumumab: | - | - | - | + | + | + | + | + | + |
| CD38-mFc: | - | - | - | - | - | - | + | + | + |
| anti-D: | + | + | + | + | + | + | + | + | + |
| ID-Dia cell type: | I | II | III | I | II | III | I | II | III |
| D phenotype: | + | + | - | + | + | - | + | + | - |
| | 1 | 2 | 3 | 4 | 5 | 6 | 7 | 8 | 9 |

FIG. 6

**B**

| | | | | | | | | | |
|---|---|---|---|---|---|---|---|---|---|
| Daratumumab: | - | - | - | + | + | + | + | + | + |
| CD38-mFc: | - | - | - | - | - | - | + | + | + |
| anti-K: | + | + | + | + | + | + | + | + | + |
| ID-Dia cell type: | I | II | III | I | II | III | I | II | III |
| K phenotype: | + | - | - | + | - | - | + | - | - |

1
2
3
4
5
6
7
8
9

FIG. 6 (continued)

Patient sample: + + + + + + + + +
CD38-mFc: - - - + + + - - -
N1-N2B-mFc: - - - - - - + + +
Cell type: I II III I II II I II III
1 2 3 4 5 6 7 8 9

FIG. 7

| | | | | | | | | | |
|---|---|---|---|---|---|---|---|---|---|
| Daratumumab: | - | - | - | + | + | + | + | + | + |
| CD38-mFc: | - | - | - | - | - | - | - | - | - |
| Grifols sCD38: | - | - | - | - | - | - | - | - | - |
| PBS volume control: | - | - | - | - | - | - | + | + | + |
| ID-Dia cell type: | I | II | III | I | II | III | I | II | III |

| | | | | | | |
|---|---|---|---|---|---|---|
| Daratumumab: | + | + | + | + | + | + |
| CD38-mFc: | + | + | + | - | - | - |
| Grifols sCD38: | - | - | - | + | + | + |
| PBS volume control: | - | - | - | - | - | - |
| ID-Dia cell type: | I | II | III | I | II | III |

FIG. 8

| | | | | | | | | | |
|---|---|---|---|---|---|---|---|---|---|
| Daratumumab: | - | - | - | + | + | + | + | + | + |
| anti-Fya: | + | + | + | + | + | + | + | + | + |
| CD38-mFc: | - | - | - | - | - | - | - | - | - |
| Grifols sCD38: | - | - | - | - | - | - | - | - | - |
| PBS volume control: | - | - | - | - | - | - | + | + | + |
| ID-Dia cell type: | I | II | III | I | II | III | I | II | III |
| Fya phenotype: | + | + | - | + | + | - | + | + | - |

| | | | | | | |
|---|---|---|---|---|---|---|
| Daratumumab: | + | + | + | + | + | + |
| anti-Fya: | + | + | + | + | + | + |
| CD38-mFc: | + | + | + | - | - | - |
| Grifols sCD38: | - | - | - | + | + | + |
| PBS volume control: | - | - | - | - | - | - |
| ID-Dia cell type: | I | II | III | I | II | III |
| Fya phenotype: | + | + | - | + | + | - |

FIG. 9

A
Recipient
Donor
Test result
Duffy antigen
Agglutination
Recipient serum with alloantibodies against Duffy antigen.
Donor RBC with Duffy antigen.

FIG. 10

B
Recipient
Donor
Test result
CD38
Agglutination
Recipient serum with therapeutic antibodies against CD38.
Donor RBC with CD38.

C
Recipient
Donor
Test result
CD38
Donor RBC with CD38.
Trypsin / DTT
No Agglutination
Recipient serum with therapeutic antibodies against CD38.

FIG. 10 (continued)

D
Recipient
Donor
Test result
CD38
Duffy antigen
Donor RBC with CD38 and Duffy antigen.
Trypsin / DTT
Recipient serum with both therapeutic antibodies against CD38 and anti-Duffy alloantibodies.
No Agglutination, although the final donor blood comprises Duffy antigen.
= FALSE NEGATIVE!

FIG. 10 (continued)

E

**Recipient** **Donor** **Test result**

Recipient serum with therapeutic antibodies against CD38.

sCD38

CD38

No Agglutination

Donor RBC with CD38.

FIG. 10 (continued)

F

Recipient

Donor

Test result

Recipient serum with both therapeutic antibodies against CD38 and anti-Duffy alloantibodies.

sCD38

CD38

Duffy antigen

Donor RBC with CD38 and Duffy antigen.

No Agglutination, although the final donor blood comprises Duffy antigen.
= FALSE NEGATIVE!

FIG. 10 (continued)

G
Recipient
Donor
Test result
Recipient serum with both therapeutic antibodies against CD38 and anti-Duffy alloantibodies.
CD38-Fc
CD38
Duffy antigen
Agglutination based on anti-Duffy reaction.
= Correct result!
Specific blockade only of anti-CD38
Donor RBC with CD38 and Duffy antigen.

FIG. 10 (continued)

Europäisches
Patentamt
European
Patent Office
Office européen
des brevets

# EUROPEAN SEARCH REPORT

Application Number
EP 24 21 5209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/030581 A1 (GRIFOLS DIAGNOSTIC SOLUTIONS INC [US]) 14 February 2019 (2019-02-14) * page 1, line 25 - page 26, line 2; claims 1-40; sequence 13 * ----- | 1-15 | INV. C07K14/705 |
| A | WO 2023/094696 A1 (MORPHOSYS AG [DE]) 1 June 2023 (2023-06-01) * page 3, line 30 - page 16, line 7 * ----- | 1-15 | |
| A | NEDUMCHERIL MARILYN T ET AL: "Overcoming Drug Interference in Transfusion Testing: A Spotlight on Daratumumab", JOURNAL OF BLOOD MEDICINE, vol. Volume 12, 25 May 2021 (2021-05-25), pages 327-336, XP055923502, DOI: 10.2147/JBM.S213510 Retrieved from the Internet: URL:https://www.dovepress.com/getfile.php?fileID=69779> ----- | 1-15 | |
| | | | TECHNICAL FIELDS SEARCHED (IPC) |
| A | MEI ZHEN ET AL: "Impact of Novel Monoclonal Antibody Therapeutics on Blood Bank Pretransfusion Testing", HEMATOLOGY - ONCOLOGY CLINICS OF NORTH AMERICA, vol. 33, no. 5, 1 October 2019 (2019-10-01), pages 797-811, XP055849125, US ISSN: 0889-8588, DOI: 10.1016/j.hoc.2019.05.007 Retrieved from the Internet: URL:http://dx.doi.org/10.1016/j.hoc.2019.05.007> * the whole document * ----- | 1-15 | C07K G01N |

~~The present search report has been drawn up for all claims~~

1

| Place of search | Date of completion of the search | Examiner |
|---|---|---|
| The Hague | 25 April 2025 | Westphal-Daniel, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt
European
Patent Office
Office européen
des brevets

**Application Number**

**EP 24 21 5209**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

## LACK OF UNITY OF INVENTION SHEET B

Application Number
EP 24 21 5209

The Search Division considers that the present European patent application does not comply with the requirements of unity of invention and relates to several inventions or groups of inventions, namely:

1. claims: 1-15(partially)

A fusion protein for binding to an agglutinating antibody, which has at least 97% sequence identity to SEQ ID NO: 19. An analytical, diagnostic or pharmaceutical composition comprising said fusion protein and further comprising at least one compound selected from the group consisting of a buffer, a stabilizer, a diluent, a filler, a binder, a disintegrant, a glidant, a preservative, a solvent, a surfactant or a combination thereof.
Use of said fusion protein or said composition for the prevention of agglutination mediated by the cross-reaction of an anti-CD38 with CD38 and/or anti-CD47-antibody with CD47 in a blood sample.
Said fusion protein or said composition for use in a method of treating a patient as set forth in claim 13.
A method for neutralizing or blocking of an anti-CD38 and/or anti-CD47 antibody in a sample comprising providing a sample with said fusion protein or a volume of said composition comprising an amount of said fusion protein sufficient to specifically neutralize the anti-CD38 and/or CD47 antibody in the sample.
A kit for biomonitoring research and diagnostic assays, comprising said fusion protein or said composition, a plate and reagents for identifying the presence of antibodies.

---

2-324. claims: 1-15(partially)

As invention 1, wherein the fusion protein for binding to an agglutinating antibody has at least 97% sequence identity to SEQ ID NO: 20 (invention 2) - SEQ ID NO: 324 (invention 324).

---

# ANNEX TO THE EUROPEAN SEARCH REPORT ON EUROPEAN PATENT APPLICATION NO.

EP 24 21 5209

This annex lists the patent family members relating to the patent documents cited in the above-mentioned European search report. The members are as contained in the European Patent Office EDP file on
The European Patent Office is in no way liable for these particulars which are merely given for the purpose of information.

25-04-2025

| Patent document cited in search report | Publication date | Patent family member(s) | Publication date |
|---|---|---|---|
| WO 2019030581 A1 | 14-02-2019 | AR 112357 A1 | 23-10-2019 |
| | | AU 2018313028 A1 | 20-02-2020 |
| | | AU 2023204121 A1 | 20-07-2023 |
| | | BR 112020002636 A2 | 28-07-2020 |
| | | CA 3070789 A1 | 14-02-2019 |
| | | CN 109790210 A | 21-05-2019 |
| | | CN 117820498 A | 05-04-2024 |
| | | EA 202090265 A1 | 05-06-2020 |
| | | EP 3464343 A1 | 10-04-2019 |
| | | EP 3587445 A1 | 01-01-2020 |
| | | EP 4296680 A2 | 27-12-2023 |
| | | ES 2911243 T3 | 18-05-2022 |
| | | ES 2973864 T3 | 24-06-2024 |
| | | HU E065510 T2 | 28-05-2024 |
| | | JP 6833851 B2 | 24-02-2021 |
| | | JP 7289328 B2 | 09-06-2023 |
| | | JP 2019527533 A | 03-10-2019 |
| | | JP 2021088567 A | 10-06-2021 |
| | | JP 2023041802 A | 24-03-2023 |
| | | KR 20200035194 A | 02-04-2020 |
| | | KR 20240015159 A | 02-02-2024 |
| | | PL 3587445 T3 | 29-04-2024 |
| | | PT 3587445 T | 14-03-2024 |
| | | TW 201910349 A | 16-03-2019 |
| | | US 2021198376 A1 | 01-07-2021 |
| | | US 2024092929 A1 | 21-03-2024 |
| | | US 2024132616 A1 | 25-04-2024 |
| | | UY 37757 A | 30-11-2018 |
| | | WO 2019030581 A1 | 14-02-2019 |
| WO 2023094696 A1 | 01-06-2023 | TW 202328207 A | 16-07-2023 |
| | | WO 2023094696 A1 | 01-06-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Antibodies against Duffy antigens may react weaker or negatively after Grifols sCD38 treatment*, hftps://www.diagnostic.grifols.com/documents/5952084/5955218/Brochure Grifols sCD381.pdf/ee5c7720-e332-646a-7e40-74f660bd3780?t=1705422448428